# EUROPÄISCHE PATENTSCHRIFT

(11) **EP 3 921 013 B1**
(45) Veröffentlichungstag und Bekanntmachung des Hinweises auf die Patenterteilung: **28.05.2025**
(21) Anmeldenummer: 20717601.7
(22) Anmeldetag: 02.04.2020
(51) Int. Cl.: A61M 39/18, F16L 37/30

(54) **STERILVERBINDER FÜR DEN STERILEN TRANSFER EINES FLÜSSIGEN MEDIUMS**
STERILE CONNECTOR FOR STERILE TRANSFER OF A LIQUID MEDIA
CONNECTEUR STERILE POUR LE TRANSFER D'UN MEDIUM LIQUIDE

(30) Priorität: 03.04.2019 DE 102019108664
(43) Veröffentlichungstag der Anmeldung: 15.12.2021
(73) Patentinhaber: Sartorius Stedim Biotech GmbH, 37079 Göttingen (DE)
(72) Erfinder: DEUSE, Mario, 37120 Bovenden (DE)
(74) Vertreter: Novagraaf International SA
(86) Internationale Anmeldenummer: PCT/EP2020/059437
(87) Internationale Veröffentlichungsnummer: WO 2020/201445

(56) Entgegenhaltungen:
- US-A1- 2009 182 309
- US-A1- 2016 022 979
- US-A1- 2019 011 070

## Beschreibung

Die Erfindung betrifft einen Sterilverbinder für den sterilen Transfer eines flüssigen, insbesondere biologischen, Mediums gemäß dem Oberbegriff von Anspruch 1, eine Koppeleinrichtung eines Sterilverbinders gemäß dem Oberbegriff von Anspruch 14, eine Verpackungsanordnung mit einer Verpackung und mindestens einer solchen darin steril verpackten Koppeleinrichtung oder einem solchen darin steril verpackten Sterilverbinder gemäß Anspruch 15 sowie die Verwendung eines solchen steril verpackten Sterilverbinders, einer solchen steril verpackten Koppeleinrichtung und/oder einer solchen Verpackungsanordnung gemäß Anspruch 17.

Unter einer bioprozesstechnischen Anlage wird hier ganz allgemein eine Einrichtung verstanden, mit der biotechnologische Prozesse durchgeführt oder unterstützt werden können. Lediglich beispielhaft seien hier Bioreaktoren genannt, in denen Mikroorganismen oder Gewebezellen unter vorgegebenen Bedingungen kultiviert werden. Eine solche Einrichtung weist regelmäßig einen Behälter auf, in welchem ein biologisches Reaktionsmedium aus den für einen biotechnologischen Prozess vorgesehenen Stoffen, z.B. Mikroorganismen oder Gewebezellen einerseits und ein entsprechendes Nährmedium andererseits, aufgenommen wird, um den jeweiligen biotechnologischen Verfahrensschritt, beispielsweise eine Fermentierung bzw. Kultivierung, durchführen zu können.

Ein Beispiel für einen solchen Bioreaktor ist zum einen ein Produktions-Bioreaktor, also ein relativ großer Bioreaktor im Produktionsmaßstab mit einem Arbeitsvolumen von beispielsweise mehreren 100 oder mehreren 1000 Litern, zur industriellen Herstellung mikrobieller oder zellulärer Produkte, insbesondere Biopharmazeutika. Ein solcher Bioreaktor bildet als Erzeugnis eine Fermentationsbrühe, die üblicherweise in einem sogenannten Downstream-Prozess weiterbehandelt wird, um ein Produkt aus den Zellen oder dem Kulturüberstand zu erhalten. Ein anderes Beispiel ist ein Labor-Bioreaktor, also ein relativ kleiner Bioreaktor im Labormaßstab mit einem Arbeitsvolumen von beispielsweise weniger als 10 Litern. Ein solcher Labor-Bioreaktor dient beispielsweise der Herstellung von ATMPs (Advanced Therapy Medical Products, Arzneimittel für neuartige Therapien) und/oder der Durchführung einer Zellexpansion, durch die eine für den jeweiligen Anwendungszweck ausreichende Anzahl von Zellen, insbesondere Gewebezellen oder mikrobiellen Zellen, erzeugt werden kann. Ein Anwendungsfall hierfür ist die Vermehrung menschlicher Zellen, beispielsweise von T-Zellen (T-Lymphozyten), die dem Patienten entnommen, anschließend ex vivo expandiert und dann dem Patienten reinfundiert werden.

Speziell bei der Herstellung von ATMPs mit einem Labor-Bioreaktor, aber grundsätzlich auch bei einem Produktions-Bioreaktor, kommt es in besonderem Maße auf eine sterile Zugabe der einzelnen flüssigen, beispielsweise biologischen, Medien in den jeweiligen Bioreaktor an. Da viele ATMPs nicht steril gefiltert oder abschließend sterilisiert werden können, erfolgt die Herstellung solcher Arzneimittel in steriler Weise in Reinräumen entsprechender Reinraumklassen. So birgt die Zugabe eines flüssigen Mediums in einen Bioreaktor oder der Transfer eines flüssigen Mediums zwischen zwei Kulturgefäßen und/oder Bioreaktoren grundsätzlich das Risiko einer Kontamination, weshalb solche Handhabungsschritte üblicherweise in einem Reinraum der Reinraumklasse A (GMP-Leitfaden Annex 1) oder ISO 5 (ISO 14644-1) durchgeführt werden. Die Herstellung von Arzneimitteln in solchen Reinräumen ist aber sehr kostenintensiv aufgrund von hohen Überwachungsanforderungen, aufwendigen Bekleidungsprozeduren etc.

Aus diesem Grund besteht die Nachfrage nach geschlossenen Systemen zur Kultivierung bzw. Expansion von Zellen, insbesondere auf dem Gebiet der Herstellung von ATMPs, wodurch Reinräume niedrigerer Reinraumklassen genutzt werden können, beispielsweise Reinräume der Reinraumklasse D (GMP-Leitfaden Annex 1) oder ISO 8 (ISO 14644-1). Eine Herausforderung ist es dabei allerdings, ein flüssiges Medium einem solchen geschlossenen System auf möglichst einfache Weise zuzugeben. Es sind zwar verschiedene Verbindungssysteme wie Luer-Verbinder, Luer-Lock-Verbinder etc. bekannt (US 2009/182309 A1), um beispielsweise einen Mindermengen-Flüssigkeitsbehälter wie eine Spritze oder Phiole mit einem geschlossenen System zu koppeln. Hierbei besteht aber an der Koppelstelle, an der die beiden Koppeleinrichtungen des Verbinders, bei einem Luer-Verbinder der männliche Luer-Anschluss und der weibliche Luer-Anschluss, miteinander verbunden werden, ein Kontaminationsrisiko, weshalb bei der Herstellung der Verbindung in besonderem Maße auf sterile Bedingungen geachtet werden muss (US 2016/022979 A1). US 2019/011070 A1 offenbart einen weiteren Konnektor aus dem Stand der Technik.

Der Erfindung liegt das Problem zugrunde, einen Sterilverbinder für den sterilen Transfer eines flüssigen Mediums von einem Flüssigkeitsbehälter in eine bioprozesstechnische Anlage derart auszugestalten und weiterzubilden, dass bei dem Transfer des Mediums sterile Bedingungen auf einfache Weise herstellbar sind.

Das obige Problem wird bei einem Sterilverbinder für den sterilen Transfer eines flüssigen, insbesondere biologischen, Mediums gemäß dem Oberbegriff von Anspruch 1 durch die Merkmale des kennzeichnenden Teils von Anspruch 1 gelöst.

Wesentlich ist die grundsätzliche Überlegung, einen Sterilverbinder mit zwei für den Flüssigkeitstransfer von einem Flüssigkeitsbehälter, der beispielsweise ein Minimalmengen-Flüssigkeitsbehälter ist, in ein bioprozesstechnische Anlage, die insbesondere ein Bioreaktor ist, miteinander zu verbindenden Koppeleinrichtungen zu schaffen, bei dem die Koppeleinrichtungen zunächst in einem Zustand bereitgestellt werden, in welchem eine Kontamination der für den Flüssigkeitstransfer maßgeblichen, bevorzugt sterilen, Teile durch den Anwender nicht ohne weiteres möglich ist, da diese in diesem Zustand in der Koppeleinrichtung verdeckt und dadurch vor Kontakt geschützt sind.

Speziell der Kanal der jeweiligen Koppeleinrichtung sowie bei der einen Koppeleinrichtung zumindest die Auslassöffnung und bei der anderen Koppeleinrichtung zumindest die dazu korrespondierende Einlassöffnung sind in diesem Zustand in einer vor Kontamination geschützten, bevorzugt sterilen, Stellung. Nachdem die beiden Koppeleinrichtungen dann bestimmungsgemäß miteinander verbunden sind, können diese jeweils in einen Zustand gebracht werden, in welchem die besagten für den Flüssigkeitstransfer maßgeblichen Teile aus der geschützten, bevorzugt sterilen, Stellung herausbewegt werden und so miteinander verbunden und zueinander ausgerichtet werden, dass der Flüssigkeitstransfer erfolgen kann. In diesem, den Transfer ermöglichenden Zustand sind dann die zuvor geschützte und noch sterile Auslassöffnung der einen Koppeleinrichtung und die zuvor geschützte und noch sterile Einlassöffnung der anderen Koppeleinrichtung so zueinander angeordnet, dass das flüssige Medium von der einen in die andere Koppeleinrichtung fließen kann. In diesem Zustand kommt das flüssige Medium im Bereich des Übergangs von der einen in die andere Koppelreinrichtung nur mit den zuvor geschützten und noch sterilen Abschnitten der Koppeleinrichtungen in Kontakt. Bereiche, die vorher ungeschützt und entsprechend einem möglichen Kontaminationsrisiko ausgesetzt waren, sind von dem fluiddurchströmten Bereich beabstandet, so dass keine Keime bzw. andere Medien in das flüssige Medium gelangen können.

Besonders bevorzugt ist, wenn bereits vor dem Verbinden der beiden Koppeleinrichtungen miteinander, der Flüssigkeitsbehälter, aus dem das Medium abgegeben werden soll, und/oder die bioprozesstechnische Anlage, in die das Medium zugegeben werden soll, steril mit der jeweils zugeordneten Koppeleinrichtung verbunden sind. Dies erfolgt besonders bevorzugt bereits auf Herstellerseite. So ist es denkbar, dass der Hersteller des Flüssigkeitsbehälters diesen bereits mit der zugeordneten Koppeleinrichtung des Sterilverbinders bereitstellt, wobei der Flüssigkeitsbehälter besonders bevorzugt zu diesem Zeitpunkt schon herstellerseitig befüllt ist, wobei der Flüssigkeitsbehälter und die Koppeleinrichtung vorzugsweise im bestimmungsgemäß miteinander verbundenen Zustand, insbesondere als Single-Use-Komponenten, dem Anwender zur Verfügung gestellt werden. Entsprechendes gilt auch für die bioprozesstechnische Anlage, bei der es ebenfalls denkbar ist, dass der Hersteller der Anlage diese bereits mit der zugeordneten Koppeleinrichtung des Sterilverbinders, gegebenenfalls auch schon mit mehreren solcher Koppeleinrichtungen, bereitstellt, wobei die bioprozesstechnische Anlage und die jeweilige Koppeleinrichtung vorzugsweise im bestimmungsgemäß miteinander verbundenen Zustand, insbesondere als Single-Use-Komponenten, dem Anwender zur Verfügung gestellt werden.

Auf diese Weise sind die beiden Koppeleinrichtungen des Sterilverbinders vor dem Herstellen der Verbindung an keiner für den Flüssigkeitstransfer maßgeblichen, bevorzugt sterilen, Stelle kontaminierbar, da in dem Bereich, in dem das Medium vom Flüssigkeitsbehälter in die damit verbundene Koppeleinrichtung gelangt, sowie in dem Bereich, in dem das Medium aus der anderen Koppeleinrichtung in die bioprozesstechnische Anlage gelangt, bereits eine sterile Verbindung besteht. Die Abschnitte der Koppeleinrichtungen, über die das Medium bestimmungsgemäß von der dem Flüssigkeitsbehälter zugeordneten Koppeleinrichtung in die jeweils andere Koppeleinrichtung gelangt, werden erst nach dem Verbinden der beiden Koppeleinrichtungen miteinander aus dem geschützten, bevorzugt sterilen, Bereich der jeweiligen Koppeleinrichtung wegbewegt und miteinander zusammengebracht. Insbesondere werden diese beiden Abschnitte unmittelbar miteinander zusammengebracht, ohne dass diese bei ihrer Bewegung aus dem geschützten Bereich zu ihrer bestimmungsgemäßen Endposition mit anderen, kontaminierbaren Abschnitten der Koppeleinrichtungen oder mit dem Anwender in Kontakt kommen können. Da erst nach dem Verbinden der beiden Koppeleinrichtungen miteinander die zu diesem Zeitpunkt noch sterilen Teile der Koppeleinrichtungen bestimmungsgemäß zueinander angeordnet und miteinander in Kontakt gebracht werden, und während dieser Bewegung keine Kontamination der Teile möglich ist, kann über den vorschlagsgemäßen Sterilverbinder auf besonders einfache Weise eine sterile Verbindung zwischen einem Flüssigkeitsbehälter und einer bioprozesstechnischen Anlage hergestellt werden.

Im Einzelnen wird nun vorgeschlagen, dass sich in der Ausgangsstellung der jeweilige Kanal in einem gegenüber der Umgebung des Gehäuses hermetisch abgedichteten Bereich des Gehäuseinnenraums erstreckt.

Als "Gehäuse" wird hier ganz allgemein ein Bauteil verstanden, dass das Stellelement zumindest abschnittsweise, vorzugsweise im Wesentlichen vollständig, in zumindest einer Umfangsrichtung umgibt. Handelt es sich bei dem Stel lelement, wie im Weiteren noch erläutert wird, um ein solches, das drehbar gelagert ist, ist dieses Stellelement von dem Gehäuse zumindest bezogen auf die Rotationsachse radialer Richtung von dem Gehäuse abschnittsweise oder im Wesentlichen vollständig umgeben. Letzteres ist gerade dann bevorzugt, wenn die Stellelement-Einlassöffnungen und Stellelement-Auslassöffnungen bezogen auf die Rotationsachse radial nach außen weisen. Grundsätzlich ist es aber auch denkbar, dass die Stellelement-Einlassöffnungen und Stellelement-Auslassöffnungen parallel zur Rotationsachse weisen, wobei in diesem Fall das Stellelement vorzugsweise in axialer Richtung von dem Gehäuse zumindest abschnittsweise oder im Wesentlichen vollständig umgeben ist. Grundsätzlich kann das Stellelement auch linear bewegbar sein, wobei dann das Gehäuse das Stellelement vorzugsweise an den zur Bewegungsrichtung parallelen Seiten zumindest abschnittsweise oder im Wesentlichen vollständig umgibt, wobei in diesem Fall die Stellelement-Einlassöffnungen und die Stellelement-Auslassöffnungen der Stellelemente in eine Richtung quer zur Bewegungsrichtung gerichtet sind.

"Im Wesentlichen vollständig" bedeutet, dass das Gehäuse hier in der jeweiligen Richtung mit Ausnahme des Fluideinlasses und des Fluidauslasses vollständig geschlossen ist.

Das Gehäuse ist jedenfalls so ausgestaltet, dass es in der Ausgangsstellung des Stellelements die Stellelement-Einlassöffnung und die Stellelement-Auslassöffnung schützt und dadurch steril hält, und in der Betriebsstellung des Stellelements ermöglicht, die Stellelement-Auslassöffnung der einen Koppeleinrichtung, die insbesondere dem Flüssigkeitsbehälter zugeordnet ist, und die Stellelement-Einlassöffnung der anderen Koppeleinrichtung, die insbesondere der bioprozesstechnischen Anlage zugeordnet ist, miteinander in Überdeckung zu bringen.

"Überdeckung" meint hier eine teilweise oder vollständige Überlappung der beiden jeweiligen Öffnungen in Richtung parallel zu den Mittelachsen der Öffnungen.

In den Ansprüchen 2 bis 7 sind besonders bevorzugte Möglichkeiten angegeben, wie sich in der Ausgangsstellung die, bevorzugt sterilen, Stellelement-Einlassöffnungen und die, bevorzugt sterilen, Stellelement-Auslassöffnungen optimal gegenüber der Umgebung und vor Kontamination geschützt in den Gehäusen anordnen lassen und wie sich diese unter Beibehaltung der Sterilität in der Betriebsstellung zueinander anordnen lassen können. Dazu ist in dem jeweiligen Gehäuse insbesondere ein hermetisch abgedichteter, vorzugsweise im Auslieferzustand bereits steriler, Bereich vorgesehen, in dem sich der durch das Stellelement hindurch verlaufende Kanal in der Ausgangstellung des Stellelements erstreckt. Erst durch eine Bewegung des Stellelements von seiner Ausgangsstellung in seine Betriebsstellung würde der Kanal und entsprechend dessen Stellelement-Einlassöffnung bzw. Stellelement-Auslassöffnung in einen nicht-sterilen Bereich gelangen. Jedoch werden bei bestimmungsgemäßer Anwendung des Sterilverbinders die beiden Koppeleinrichtungen vorher so miteinander verbunden, dass die Bewegung des Stellelements von der Ausgangstellung in die Betriebsstellung zu keinem Kontakt der Stellelement-Einlassöffnung, der Stellelement-Auslassöffnung und des Kanals mit der Umgebung führen kann.

"Hermetisch dicht" bzw. "hermetisch abgedichtet" meint in diesem Zusammenhang, dass eine Dichtigkeit bereitgestellt wird, die ein Eindringen von Verunreinigungen, insbesondere Keimen, verhindert. In einer weiteren Ausführungsform bezieht sich "hermetisch dicht" bzw. "hermetisch abgedichtet" auf einen sterilen und/oder vor Kontamination geschützten Bereich des Sterilverbinders. In einer besonders bevorzugten Ausführungsform umfassen die Begriffe "hermetisch dicht" bzw. "hermetisch abgedichtet" ebenfalls den Begriff "steril" und sind somit austauschbar.

Anspruch 8 betrifft besonders bevorzugte Ausrichtungen aller fluiddurchströmten Teile in der Betriebsstellung des jeweiligen Stellelements. Besonders bevorzugt verlaufen hierbei die beiden Fluideinlässe der Gehäuse, die beiden Fluidauslässe der Gehäuse und die beiden Kanäle der Stellelemente koaxial zueinander. Grundsätzlich sind hier aber auch andere Verläufe denkbar.

Die weiter bevorzugten Ausgestaltungen in den Ansprüchen 9 bis 11 betreffen die Verstellbewegung des jeweiligen Stellelements zwischen der Ausgangsstellung und der Betriebsstellung. Insbesondere geht die Verstellbewegung des einen Stellelements mit einer korrespondierenden Verstellbewegung des anderen Stellelements einher, das heißt, beide Stellelemente bewegen sich synchron und befinden sich gleichzeitig in ihrer Ausgangsstellung und gleichzeitig in ihrer Betriebsstellung. Dies wird vorzugsweise über ein Getriebe, insbesondere Zahnradgetriebe, ermöglicht. Das Getriebe kann vom Anwender von außerhalb der Gehäuse betätigt werden, wodurch die Verstellbewegungen beider Stellelemente bewirkt werden.

Nach der besonders bevorzugten Ausgestaltung gemäß Anspruch 12 ist der Fluideinlass des einen Gehäuses und der Fluidauslass des anderen Gehäuses mit einem Schlauch oder Röhrchen verbindbar oder verbunden. Wie zuvor bereits erläutert, ist es besonders bevorzugt, wenn bereits herstellerseitig der Flüssigkeitsbehälter mit der jeweils zugeordneten, einen Koppeleinrichtung und die bioprozesstechnische Anlage mit der jeweils zugeordneten, anderen Koppeleinrichtung verbunden ist, was ebenfalls über einen entsprechenden Schlauch oder ein entsprechendes Röhrchen erfolgen kann.

Anspruch 13 definiert bevorzugte Möglichkeiten der Verbindung der beiden Gehäuse miteinander.

Nach einer weiteren Lehre gemäß Anspruch 14, der eigenständige Bedeutung zukommt, wird eine Koppeleinrichtung eines Sterilverbinders, insbesondere eines vorschlagsgemäßen Sterilverbinders, für den sterilen Transfer eines flüssigen, insbesondere biologischen, Mediums von einem Flüssigkeitsbehälter in eine bioprozesstechnische Anlage, insbesondere in einen Bioreaktor, beansprucht. Angesichts der Tatsache, dass der vorschlagsgemäße Sterilverbinder zwei Koppeleinrichtungen aufweist, darf auf alle diesbezüglichen Ausführungen zu der erstgenannten Lehre verwiesen werden.

Nach einer weiteren Lehre gemäß Anspruch 15, der ebenfalls eigenständige Bedeutung zukommt, wird eine Verpackungsanordnung mit einer Verpackung und mit mindestens einer, vorzugsweise genau einer, darin steril verpackten, vorschlagsgemäßen Koppeleinrichtung oder einem darin steril verpackten, vorschlagsgemäßen Sterilverbinder beansprucht. Angesichts der Tatsache, dass die vorschlagsgemäße Verpackungsanordnung einen vorschlagsgemäßen Sterilverbinder oder eine vorschlagsgemäße Koppeleinrichtung aufweist, darf auf alle diesbezüglichen Ausführungen zu der erstgenannten und der zweitgenannten Lehre verwiesen werden.

Gemäß Anspruch 16 kann in der Verpackung außerdem entweder ein Flüssigkeitsbehälter oder eine bioprozesstechnische Anlage steril verpackt sein, wobei der Flüssigkeitsbehälter bzw. die bioprozesstechnische Anlage mit der Koppeleinrichtung in der Verpackung bereits fluidtechnisch gekoppelt ist.

Nach einer weiteren Lehre gemäß Anspruch 17, der ebenfalls eigenständige Bedeutung zukommt, wird die Verwendung eines steril verpackten, vorschlagsgemäßen Sterilverbinders, einer steril verpackten, vorschlagsgemäßen Koppeleinrichtung und/oder einer vorschlagsgemäßen Verpackungsanordnung für den sterilen Transfer eines flüssigen, insbesondere biologischen, Mediums von einem Flüssigkeitsbehälter in eine bioprozesstechnische Anlage, insbesondere in einem Bioreaktor, beansprucht. Angesichts der Tatsache, dass die vorschlagsgemäße Verwendung die Verwendung eines vorschlagsgemäßen Sterilverbinders, einer vorschlagsgemäßen Koppeleinrichtung und/oder einer vorschlagsgemäßen Verpackungsanordnung betrifft, darf auf alle diesbezüglichen Ausführungen zu den vorgenannten Lehren verwiesen werden.

Mit der vorschlagsgemäßen Verpackungsanordnung und der vorschlagsgemäßen Verwendung ist erkannt worden, dass sich mit dem vorschlagsgemäßen Sterilverbinder bzw. mit den vorschlagsgemäßen Koppeleinrichtungen ein flüssiges Medium besonders einfach ohne nennenswertes Risiko einer Kontamination einem geschlossenen System, insbesondere einem Bioreaktor, zuführen lässt, indem die Koppeleinrichtungen die für den Flüssigkeitstransfer maßgeblichen Teile so lange in einem sterilen Zustand halten, nämlich in besagter Ausgangsstellung, bis der Flüssigkeitstransfer, nämlich in besagter Betriebsstellung, tatsächlich erfolgt.

Bei der besonders bevorzugten Ausgestaltung gemäß Anspruch 18 sind einzelne der genannten Komponenten, insbesondere die vorschlagsgemäße Koppeleinrichtung, insbesondere beide Koppeleinrichtungen, vorzugsweise auch der Flüssigkeitsbehälter und/oder die bioprozesstechnische Anlage und/oder der oder die Schläuche oder Röhrchen vorzugsweise als Single-Use-Komponenten ausgestaltet, wobei die Komponenten vorzugsweise aus einem Kunststoffmaterial ausgelegt sind.

Im Folgenden wird die Erfindung anhand einer lediglich ein Ausführungsbeispiel darstellenden Zeichnung näher erläutert. In der Zeichnung zeigt
- **Fig. 1**: **in einer perspektivischen Ansicht einen vorschlagsgemäßen Sterilverbinder beim Verbinden seiner beiden Koppeleinrichtungen,**
- **Fig. 2**: **in einer perspektivischen Ansicht die beiden Koppeleinrichtungen im verbundenen Zustand a) in der Ausgangsstellung der beiden Stellelemente und b) in der Betriebsstellung der beiden Stellelemente,**
- **Fig. 3**: **in einer geschnittenen Ansicht die beiden Koppeleinrichtungen im verbundenen Zustand a) in der Ausgangsstellung der beiden Stellelemente und b) in der Betriebsstellung der beiden Stellelemente und**
- **Fig. 4**: **in einer perspektivischen Ansicht eine vorschlagsgemäße Verpackungsanordnung mit einer vorschlagsgemäßen Koppeleinrichtung und einem daran angeschlossenen Flüssigkeitsbehälter sowie eine vorschlagsgemäße Verpackungsanordnung mit einer weiteren vorschlagsgemäßen Koppeleinrichtung und einer daran angeschlossenen bioprozesstechnischen Anlage.**

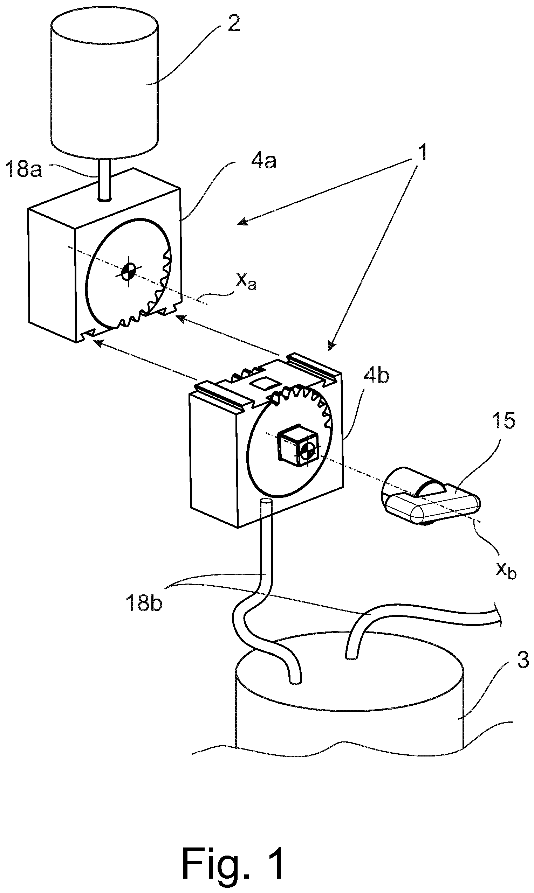

Der in den Figuren 1 bis 4 dargestellte, vorschlagsgemäße Sterilverbinder 1 dient zum sterilen Transfer eines flüssigen, insbesondere biologischen, Mediums von einem Flüssigkeitsbehälter 2, hier einem Mindermengen-Flüssigkeitsbehälter, also einem Flüssigkeitsbehälter mit geringem Aufnahmevolumen von beispielsweise maximal 30 ml, in eine bioprozesstechnische Anlage 3, hier in einen Bioreaktor. Der vorschlagsgemäße Sterilverbinder 1 weist zwei miteinander mechanisch und fluidtechnisch verbindbare Koppeleinrichtungen 4a, 4b auf, von denen die eine Koppeleinrichtung 4a dem Flüssigkeitsbehälter 2 und die andere Koppeleinrichtung 4b der bioprozesstechnischen Anlage 3 zugeordnet ist. "Zugeordnet" meint, dass die Koppeleinrichtung 4a mit dem Flüssigkeitsbehälter 2 verbindbar oder verbunden ist und die Koppeleinrichtung 4b mit der bioprozesstechnischen Anlage 3 verbindbar oder verbunden ist. Besonders bevorzugt ist dabei bereits herstellerseitig, und zwar im sterilen Zustand, zum einen der Flüssigkeitsbehälter 2 mit der diesem zugeordneten Koppeleinrichtung 4a und die bioprozesstechnische Anlage 3 mit der dieser zugeordneten Koppeleinrichtung 4b verbunden worden.

Die bioprozesstechnische Anlage 3, insbesondere der Bioreaktor, bildet hier ein geschlossenes System oder ist in ein geschlossenes System integriert. Der Bioreaktor ist beispielsweise als Labor-Bioreaktor ausgestaltet, also als ein Bioreaktor, der im Unterschied zu einem Produktions-Bioreaktor ein relativ kleines Arbeitsvolumen (maximal zur Verfügung stehendes Füllvolumen) von maximal 10 Litern aufweist. Ein solcher Bioreaktor dient der Durchführung eines biotechnologischen Prozesses der hier und vorzugsweise zur Herstellung eines Arzneimittels für neuartige Therapien (ATMP) dient. Beispielsweise kann der biotechnologische Prozess auch ein Zellexpansionsprozess für T-Zellen sein. Jedenfalls wird im Bioreaktor ein biologisches Reaktionsmedium vorgesehen, dass insbesondere Gewebezellen oder mikrobielle Zellen sowie ein Nährmedium aufweist. Hierbei ist es notwendig, die flüssigen Medien dem geschlossenen System steril zuzugeben.

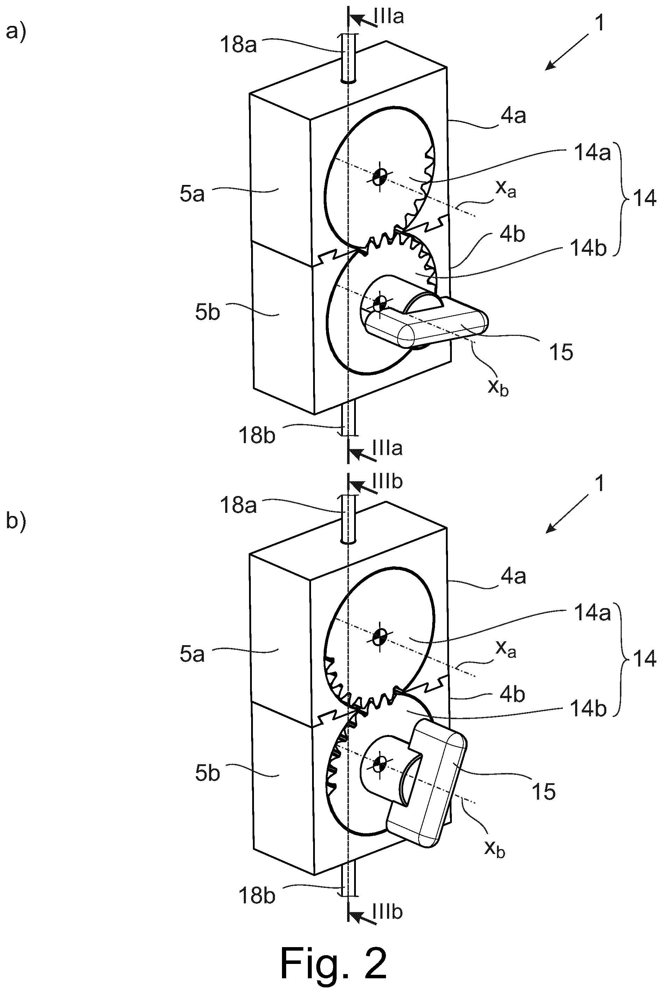

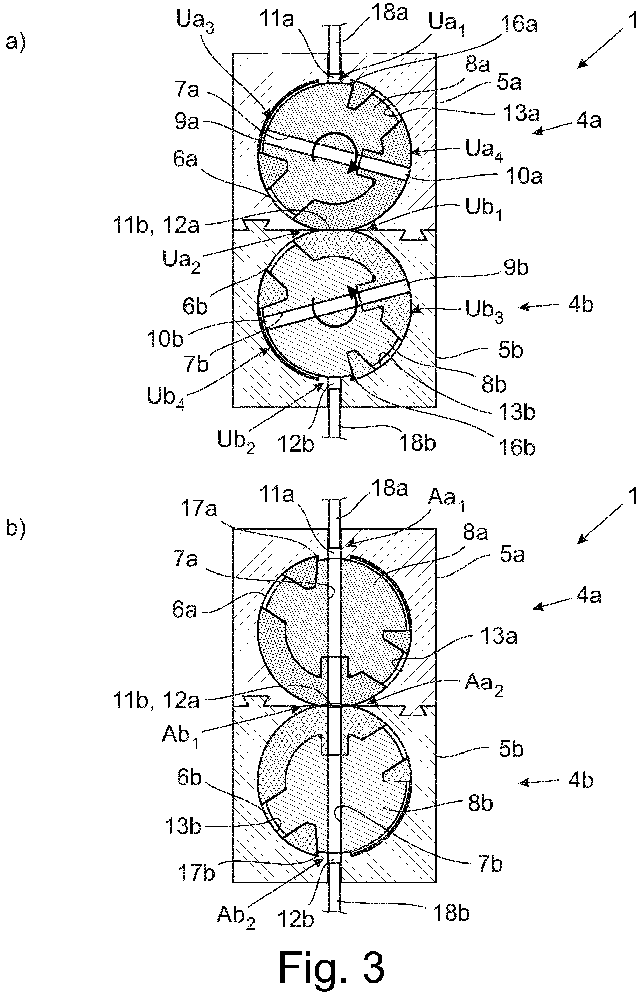

Die Koppeleinrichtungen 4a, 4b weisen jeweils ein Gehäuse 5a, 5b mit einem, bevorzugt sterilen, Gehäuseinnenraum 6a, 6b und ein im Gehäuseinnenraum 6a, 6b verstellbar gelagertes, einen Kanal 7a, 7b bildendes, bevorzugt steriles, Stellelement 8a, 8b auf, wobei sich der Kanal 7a, 7b von einer Stellelement-Einlassöffnung 9a, 9b zu einer Stellelement-Auslassöffnung 10a, 10b durch das Stellelement 8a, 8b hindurch erstreckt. Weiter ist Wesentlich, dass das Gehäuse 5a der einen Koppeleinrichtung 4a mit dem Gehäuse 5b der anderen Koppeleinrichtung 4b mechanisch verbindbar ist. Auch ist wesentlich, dass das jeweilige Gehäuse 5a, 5b einen Fluideinlass 11a, 11b und einen Fluidauslass 12a, 12b aufweist, die den Gehäuseinnenraum 6a, 6b mit der Umgebung des jeweiligen Gehäuses 5a, 5b verbinden, wobei im mechanisch miteinander verbundenen Zustand der Gehäuse 5a, 5b der Fluidauslass 12a des einen Gehäuses 5a und der Fluideinlass 11b des anderen Gehäuses 5b miteinander in Überdeckung sind. Schließlich ist auch wesentlich, dass im mechanisch miteinander verbundenen Zustand der Gehäuse 5a, 5b die Stellelemente 8a, 8b jeweils von einer Ausgangsstellung (Fig. 2a), Fig. 3a)), in der eine Fluidverbindung zwischen Kanal 7a, 7b und Stellelement-Einlassöffnung 9a, 9b einerseits und zwischen Kanal 7a, 7b und Stellelement-Auslassöffnung 10a, 10b andererseits blockiert ist, in eine Betriebsstellung (Fig. 2b), Fig. 3b)), in der die Stellelement-Einlassöffnung 9a, 9b mit dem Fluideinlass 11a, 11b und die Stellelement-Auslassöffnung 10a, 10b mit dem Fluidauslass 12a, 12b, insbesondere vollständig, in Fluidverbindung steht, verstellbar sind.

Wesentlich ist nun, dass sich in der Ausgangsstellung der jeweilige Kanal 7a, 7b in einem gegenüber der Umgebung des Gehäuses 5a, 5b hermetisch abgedichteten Bereich des Gehäuseinnenraums 6a, 6b erstreckt.

Bei dem vorschlagsgemäßen Sterilverbinder 1 bzw. den vorschlagsgemäßen, miteinander zu verbindenden Koppeleinrichtungen 4a, 4b ist also vorgesehen, dass die Stellelemente 8a, 8b von einer Ausgangsstellung in eine Betriebsstellung bewegt werden können, wobei in der Ausgangstellung die für den Fluidtransfer maßgeblichen Teile, nämlich der Kanal 7a, 7b, die Stellelement-Einlassöffnung 9a, 9b und/oder die Stellelement-Auslassöffnung 10a, 10b, in einem sterilen Zustand vorliegen. Der sterile Zustand wird dadurch gewährleistet, dass die für den Fluidtransfer maßgeblichen Teile in der Ausgangsstellung des Stellelements 8a, 8b bevorzugt steril vorliegen und gegenüber der Umgebung des Gehäuses 5a, 5b abgedeckt und entsprechend vor Kontamination geschützt sind. Sind die beiden Koppeleinrichtungen 4a, 4b miteinander bestimmungsgemäß verbunden, lassen sich die Stellelemente 8a, 8b in die Betriebsstellung bewegen, in der dann der Transfer des flüssigen Mediums erfolgen kann. In der Betriebsstellung sind die besagten, für den Fluidtransfer maßgeblichen Teile nach wie vor steril, da die beiden Koppeleinrichtungen 4a, 4b bzw. die beiden Gehäuse 5a, 5b vorher, also vor der Bewegung von der Ausgangsstellung in die Betriebsstellung, so miteinander verbunden worden sind, dass die für den Fluidtransfer maßgeblichen Teile zu keinem Zeitpunkt mit der Umgebung in Kontakt kommen konnten.

Bei dem hier dargestellten und insoweit bevorzugten Ausführungsbeispiel erstreckt sich in der Ausgangsstellung der jeweilige Kanal 7a, 7b in einem gegenüber der Umgebung des Gehäuses 5a, 5b im Auslieferungszustand bereits sterilen Bereich des Gehäuseinnenraums 6a, 6b. Hier und vorzugsweise ist es weiter so, dass in der Ausgangsstellung die Stellelement-Einlassöffnung 9a, 9b und die Stellelement-Auslassöffnung 10a, 10b jeweils zu der Gehäuseinnenwand 13a, 13b des jeweiligen Gehäuses 5a, 5b weisen.

Bei dem jeweiligen Stellelement 8a, 8b handelt es sich hier und vorzugsweise um ein solches, das drehbar gelagert ist und, wie Fig. 3 zeigt, einen im Wesentlichen runden Querschnitt hat. Entsprechend weist das jeweilige Stellelement 8a, 8b einen um die Rotationsachse xₐ, x_{b} des Stellelements 8a, 8b umlaufenden, im Wesentlichen zylindrischen Umfang auf, wobei die jeweilige Stellelement-Einlassöffnung 9a, 9b und die jeweilige Stellelement-Auslassöffnung 10a, 10b hier und vorzugsweise an dem Umfang angeordnet sind und in radialer Richtung bezogen auf die Rotationsachsen xₐ, x_{b} weisen. Der die jeweilige Stellelement-Einlassöffnung 9a, 9b mit der jeweiligen Stellelement-Auslassöffnung 10a, 10b verbindende Kanal 7a, 7b hat hier und vorzugsweise einen geraden Verlauf. Der Kanal verläuft hier mittig durch das jeweilige Stellelement 8a, 8b, somit also auch durch die Rotationsachse xₐ, x_{b} des jeweiligen Stellelements 8a, 8b. Die Stellelement-Einlassöffnung 9a, 9b und die Stellelement-Auslassöffnung 10a, 10b des jeweiligen Stellelements 8a, 8b liegen hier und vorzugsweise einander diametral gegenüber. In einem alternativen und hier nicht dargestellten Ausführungsbeispiel ist es auch denkbar, dass die Stellelement-Einlassöffnung und/oder Stellelement-Auslassöffnung des Stellelements 8a, 8b seitlich, das heißt parallel zur Rotationsachse xₐ, x_{b}, gerichtet sein kann. Zusätzlich oder alternativ ist es in einem weiteren, hier nicht dargestellten Ausführungsbeispiel auch denkbar, anstelle eines drehbaren Stellelements 8a, 8b ein linear bewegbares Stellelement vorzusehen. Die zu den hier und vorzugsweise drehbaren Stellelementen 8a, 8b erfolgenden Ausführungen gelten entsprechend gleichermaßen auch für anders gestaltete bzw. verstellbare Stellelemente, insbesondere auch linear verstellbare Stellelemente.

Der Fluideinlass 11a des einen Gehäuses 5a und der Fluidauslass 12b des anderen Gehäuses 5b ist hier kanalförmig, das heißt als ein von außen in den Gehäuseinnenraum 6a, 6b führender Einlass- bzw. Auslasskanal, ausgestaltet, wohingegen der Fluidauslass 12a des einen Gehäuses 5a und der Fluideinlass 11b des anderen Gehäuses 5b hier als eine Öffnung ausgestaltet ist, die den Gehäuseinnenraum 6a, 6b unmittelbar mit der Umgebung verbindet. In einer exemplarischen Ausführungsform ist der Querschnitt des Fluideinlasses 11a des einen Gehäuses 5a und des Fluidauslasses 12b des anderen Gehäuses 5b kleiner als der Querschnitt des Fluidauslasses 12a des einen Gehäuses 5a und des Fluideinlasses 11b des anderen Gehäuses 5b. Der "Querschnitt" ist dabei immer bezogen auf einen Schnitt quer zur Strömungsrichtung. Die Längserstreckung des Fluideinlasses 11a des einen Gehäuses 5a und des Fluidauslasses 12b des anderen Gehäuses 5b ist hier außerdem größer als die Längserstreckung des Fluidauslasses 12a des einen Gehäuses 5a und des Fluideinlasses 11b des anderen Gehäuses 5b. Letztere ist nahezu null. Die "Längserstreckung" ist dabei immer bezogen auf die Strömungsrichtung.

Hier und vorzugsweise ist es nun weiter so, dass in der Ausgangsstellung ein Abschnitt, insbesondere ein Umfangsabschnitt Ua₁, Ub₁, des jeweiligen Stellelements 8a, 8b den Fluideinlass 11a, 11b des jeweiligen Gehäuses 5a, 5b hermetisch dicht verschließt. Zusätzlich oder alternativ ist hier und vorzugsweise ferner vorgesehen, dass in der Ausgangsstellung ein weiterer Abschnitt, insbesondere ein weiterer Umfangsabschnitt Ua₂, Ub₂, des jeweiligen Stellelements 8a, 8b den Fluidauslass 12a, 12b des jeweiligen Gehäuses 5a, 5b hermetisch dicht verschließt.

Weiter ist es hier und vorzugsweise so, dass in der Ausgangstellung ein um die Stellelement-Einlassöffnung 9a, 9b herum verlaufender Abschnitt, insbesondere ein Umfangsabschnitt Ua₃, Ub₃, des jeweiligen Stellelements 8a, 8b hermetisch dichtend an der Gehäuseinnenwand 13a, 13b des jeweiligen Gehäuses 5a, 5b anliegt. Zusätzlich oder alternativ ist hier und vorzugsweise vorgesehen, dass in der Ausgangsstellung ein um die Stellelement-Auslassöffnung 10a, 10b herum verlaufender Abschnitt, insbesondere ein Umfangsabschnitt Ua₄, Ub₄, des jeweiligen Stellelements 8a, 8b hermetisch dichtend an der Gehäuseinnenwand 13a, 13b des jeweiligen Gehäuses 5a, 5b anliegt.

Darüber hinaus ist es hier und vorzugsweise so, dass in der Ausgangsstellung ein um den Fluideinlass 11a herum verlaufender Abschnitt Aa₁ des Gehäuses 5a der hier dem Flüssigkeitsbehälter 2 zugeordneten Koppeleinrichtung 4a und/oder ein um den Fluidauslass 12b herum verlaufender Abschnitt Ab₂ des Gehäuses 5b der hier der bioprozesstechnischen Anlage 3 zugeordneten Koppeleinrichtung 4b hermetisch dichtend an dem jeweiligen Stellelement 8a, 8b anliegt, nämlich der Abschnitt Aa₁ an dem Stellelement 8a und der Abschnitt Ab₂ an dem Stellelement 8b. Hier und vorzugsweise ist weiter vorgesehen, dass in der Ausgangstellung ein um den Fluidauslass 12a herum verlaufender Abschnitt Aa₂ des Gehäuses 5a der hier dem Flüssigkeitsbehälter 2 zugeordneten Koppeleinrichtung 4a und/oder ein um den Fluideinlass 11b herum verlaufender Abschnitt Ab₁ des Gehäuses 5b der hier der bioprozesstechnischen Anlage 3 zugeordneten Koppeleinrichtung 4b hermetisch dichtend an dem jeweiligen Stellelement 8a, 8b anliegt.

Wie insbesondere die Figuren 3a) und b) zeigen, liegen das Stellelement 8a des einen Gehäuses 5a und das Stellelement 8b des anderen Gehäuses 5b im mechanisch miteinander verbundenen Zustand der Gehäuse 5a, 5b zumindest dann, wenn beide Stellelemente 8a, 8b in ihrer Betriebsstellung sind, hier und vorzugsweise auch dann, wenn beide Stellelemente 8a, 8b in ihrer Ausgangsstellung sind, unter Vorspannung aneinander an. Hier und vorzugsweise liegen die beiden Stellelemente 8a, 8b ständig unter Vorspannung aneinander an. Dies wird insbesondere dadurch erreicht, dass die Stellelemente 8a, 8b entweder insgesamt oder zumindest nur im Bereich ihres gegenseitigen Kontakts aus einem vergleichsweise weichen Kunststoff, insbesondere aus einem Elastomer, insbesondere aus einem Silikon-Material, ausgelegt sind. Auf diese Weise können sich die Stellelemente 8a, 8b im Bereich des gegenseitigen Kontakts elastisch verformen. Wie Fig. 3 zeigt, ist es hier und vorzugsweise so, dass im mechanisch miteinander verbundenen Zustand der Gehäuse 5a, 5b dann, wenn beide Stellelemente 8a, 8b in ihrer Betriebsstellung sind, ein um die Stellelement-Auslassöffnung 10a herum verlaufender Abschnitt, insbesondere Umfangsabschnitt Ua₄, des einen Stellelements 8a und ein um die Stellelement-Einlassöffnung 9b herum verlaufender Abschnitt, insbesondere Umfangsabschnitt Ub₃, des anderen Stellelements 8b den jeweiligen Kanal 7a, 7b zum jeweiligen Gehäuse 5a, 5b hin hermetisch abdichtend aneinander anliegen.

Zum Zwecke der hermetischen Abdichtung ist wie gesagt insbesondere ein Elastomer vorgesehen. Besonders bevorzugt ist das Stellelement 8a, 8b jeweils abschnittsweise oder vollständig aus einem solchen Elastomer, insbesondere aus einem Silikon-Material, ausgelegt. Auch das jeweilige Gehäuse 5a, 5b kann, jedenfalls abschnittsweise, aus einem Elastomer, insbesondere aus einem Silikon-Material, ausgelegt sein, obwohl bevorzugt ist, dass das Gehäuse 5a, 5b aus einem möglichst wenig elastischen Kunststoff, vorzugsweise aus einem Kunststoff mit einer geringeren Elastizität, insbesondere einem Thermoplast oder Vero-Material, ausgelegt ist. Gemäß einer besonders bevorzugten Ausgestaltung ist vorgesehen, das das jeweilige Stellelement 8a, 8b abschnittsweise oder vollständig, insbesondere in dem Abschnitt, hier Umfangsabschnitt Ua₁, Ub₁, der den Fluideinlass 11a, 11b hermetisch dicht verschließt, und/oder in dem Abschnitt, hier Umfangsabschnitt Ua₂, Ub₂, der den Fluidauslass 12a, 12b hermetisch dicht verschließt, und/oder in dem um die Stellelement-Einlassöffnung 9a, 9b herum verlaufenden Abschnitt, hier Umfangsabschnitt Uas, Ub₃, und/oder in dem um die Stellelement-Auslassöffnung 10a, 10b herum verlaufenden Abschnitt, hier Umfangsabschnitt Ua₄, Ub₄, aus einem Elastomer, insbesondere aus einem Silikon-Material, ausgelegt ist. Zusätzlich oder alternativ kann vorgesehen sein, dass das jeweilige Gehäuse 5a, 5b abschnittsweise, insbesondere in dem um den Fluideinlass 11a, 11b herum verlaufenden Abschnitt Aa₁, Ab₁ und/oder in dem um den Fluidauslass 12a, 12b herum verlaufenden Abschnitt Aa₂, Ab₂, aus einem Elastomer, insbesondere aus einem Silikon-Material, und vorzugsweise im Übrigen aus einem Kunststoff mit einer geringeren Elastizität, insbesondere einem Thermoplast oder Vero-Material, ausgelegt ist.

Wie Fig. 3b) ferner veranschaulicht, sind im mechanisch miteinander verbundenen Zustand der Gehäuse 5a, 5b, wenn beide Stellelemente 8a, 8b in ihrer Betriebsstellung sind, bei den Koppeleinrichtungen 4a, 4b jeweils der Fluideinlass 11a, 11b und die Stellelement-Einlassöffnung 9a, 9b einerseits und/oder der Fluidauslass 12a, 12b und die Stellelement-Auslassöffnung 10a, 10b andererseits miteinander in Überdeckung. Insbesondere sind diese koaxial zueinander ausgerichtet. Dabei sind gleichzeitig auch der Fluidauslass 12a des einen Gehäuses 5a und der Fluideinlass 11b des anderen Gehäuses 5b miteinander in Überdeckung. Auch diese sind hier und vorzugsweise koaxial zueinander ausgerichtet. Besonders bevorzugt sind hier, wie Fig. 3b) zeigt, die beiden Fluideinlässe 11a, 11b, die beiden Fluidauslässe 12a, 12b und die beiden Kanäle 7a, 7b koaxial zueinander ausgerichtet.

Die in Fig. 3a) durch einen umlaufenden Pfeil dargestellte Verstellbewegung des einen Stellelements 8a zwischen seiner Ausgangsstellung und seiner Betriebsstellung geht hier und vorzugsweise mit einer korrespondierenden Verstellbewegung des anderen Stellelements 8b einher. Dies gilt hier und vorzugsweise ausschließlich für den mechanisch miteinander verbundenen Zustand der Gehäuse 5a, 5b, wie dieser in den Figuren 2 und 3 dargestellt ist. Zu diesem Zweck ist hier und vorzugsweise ein Getriebe 14 vorgesehen, das, wie in Fig. 2 dargestellt, besonders bevorzugt ein Zahnradgetriebe ist. Das Getriebe 14 weist mindestens zwei Getriebekomponenten auf, vorzugsweise jeweils in Form eines Zahnrads und/oder Zahnradsegments 14a, 14b. Als Zahnradsegment 14a, 14b ist hier beispielsweise ein verzahnter Umfangsabschnitt einer kreisförmigen Scheibe vorgesehen, wobei anstelle einer wie hier vollständig umlaufenden Scheibe diese auch lediglich die Form eines Kreisausschnitts aufweisen kann, wobei sich die Verzahnung dann entlang des Kreisbogens erstreckt. Hier und vorzugsweise sind zwei Zahnradsegmente 14a, 14b als Getriebekomponenten vorgesehen. Im mechanisch miteinander verbundenen Zustand der Gehäuse 5a, 5b bewirkt das Getriebe 14 ein synchrones Verstellen der beiden Stellelemente 8a, 8b zwischen ihrer Ausgangsstellung und ihrer Betriebsstellung. Hier und vorzugsweise ist außerdem eine Getriebekomponente des Getriebes 14, insbesondere ein Zahnrad oder Zahnradsegment 14b des Getriebes 14, mit einem Betätigungselement 15 zur manuellen Betätigung des Getriebes 14 drehfest verbunden oder verbindbar. Bei dem Betätigungselement 15 kann es sich um einen Hebel, eine Kurbel oder dergleichen handeln.

Die Verstellbewegung des jeweiligen Stellelements 8a, 8b zwischen der Ausgangsstellung und der Betriebsstellung ist, wie zuvor angedeutet wurde, hier und vorzugsweise eine rotatorische Bewegung, das heißt, das Stellelement 8a, 8b ist hier jeweils ein drehbares Stellelement. Dabei wird das jeweilige Stellelement 8a, 8b hier und vorzugsweise zwischen der Ausgangsstellung und der Betriebsstellung in einem Winkel von weniger als 45 Grad, vorzugsweise von weniger als 40 Grad, weiter vorzugsweise von weniger als 35 Grad, weiter vorzugsweise von weniger als 30 Grad, verstellt. Grundsätzlich ist in einem alternativen, hier nicht dargestellten Ausführungsbeispiel wie gesagt auch eine lineare Bewegung des jeweiligen Stellelements als Verstellbewegung denkbar.

Die Verstellbewegung des jeweiligen Stellelements 8a, 8b kann in eine oder beide Richtungen, also zur Ausgangsstellung hin und/oder zur Betriebsstellung hin, begrenzt sein. Hier und vorzugsweise ist dies in beiden Richtungen dadurch gewährleistet, dass zumindest eine Getriebekomponente, hier beide Getriebekomponenten, ein Zahnradsegment 14a, 14b ist bzw. sind, sodass eine Verstellbewegung nur in dem verzahnten Umfangsabschnitt möglich ist, also beispielsweise nur in einem Winkelbereich von weniger als 45 Grad, vorzugsweise von weniger als 40 Grad, weiter vorzugsweise von weniger als 35 Grad, weiter vorzugsweise von weniger als 30 Grad. Zusätzlich oder alternativ kann, wie in den Figuren 3a) und b) lediglich beispielhaft gezeigt ist, die Verstellbewegung in der Ausgangsstellung über einen Anschlag 16a, 16b und/oder in der Betriebsstellung über einen Anschlag 17a, 17b begrenzt sein. Der jeweilige Anschlag 16a, 16b und/oder der jeweilige Anschlag 17a, 17b kann wie hier innerhalb des Gehäuses 5a, 5b oder auch außerhalb des Gehäuses 5a, 5b vorgesehen sein.

Auf diese Weise kann zunächst, insbesondere herstellerseitig, die Ausgangsstellung als definierte Stellung eingestellt werden, so dass sicher gewährleistet ist, dass sich die für den Fluidtransfer maßgeblichen Teile im geschützten und insbesondere sterilen Bereich befinden. Anschließend kann, insbesondere anwenderseitig, die Betriebsstellung als definierte Stellung eingestellt werden, so dass sicher gewährleistet ist, dass die jeweilige Stellelement-Einlassöffnung 9a, 9b mit dem jeweils zugeordneten Fluideinlass 11a, 11b und die jeweilige Stellelement-Auslassöffnung 10a, 10b mit dem jeweils zugeordneten Fluidauslass 12a, 12b in Überdeckung ist.

Die Figuren 1 und 2 zeigen ferner, dass hier und vorzugsweise der Fluideinlass 11a des einen Gehäuses 5a und der Fluidauslass 12b des anderen Gehäuses 5b, insbesondere über eine Steckverbindung, jeweils mit einem Schlauch 18a, 18b oder Röhrchen verbindbar oder verbunden ist. Hier und vorzugsweise ist der Fluideinlass 11a des einen Gehäuses 5a über einen Schlauch 18a oder ein Röhrchen mit dem Flüssigkeitsbehälter 2 gekoppelt. Zusätzlich oder alternativ ist, wie die Figuren 1 und 2 ebenfalls zeigen, der Fluidauslass 12b des anderen Gehäuses 5b über einen Schlauch 18b oder ein Röhrchen mit der bioprozesstechnischen Anlage 3, hier dem Bioreaktor, fluidtechnisch gekoppelt.

Weiterhin lassen die Figuren 1 bis 3 erkennen, dass die Gehäuse 5a, 5b formschlüssig miteinander verbindbar sind. Hier sind diese lediglich beispielhaft zusammensteckbar, beispielsweise über eine Schwalbenschwanzverbindung oder dergleichen. Grundsätzlich ist aber auch denkbar, dass die Gehäuse 5a, 5b miteinander verrastbar sind. Wesentlich ist nur, dass die Gehäuse 5a, 5b bei Durchführung der Verstellbewegung der Stellelemente 8a, 8b sicher zusammen gehalten werden. Da die Stellelemente, jedenfalls in der Betriebsstellung, vorzugsweise unter Vorspannung aneinander anliegen, besteht der Formschluss vorzugsweise ebenfalls in Verlaufsrichtung der Vorspannung. Hier und vorzugsweise besteht der Formschluss zwischen den Gehäusen 5a, 5b in Strömungsrichtung des flüssigen Mediums bei dessen Transfer.

Nach einer weiteren Lehre, der eigenständige Bedeutung zukommt, wird die jeweilige Koppeleinrichtung 4a, 4b, also hier die dem Flüssigkeitsbehälter 2 zugeordnete Koppeleinrichtung 4a und/oder die der bioprozesstechnischen Anlage 3 zugeordnete Koppeleinrichtung 4b, als solche beansprucht. Insbesondere ist die Koppeleinrichtung 4a, 4b eine Koppeleinrichtung des vorschlagsgemä-ßen Sterilverbinders 1.

Eine solche Koppeleinrichtung 4a weist ein Gehäuse 5a mit einem Gehäuseinnenraum 6a und ein im Gehäuseinnenraum 6a verstellbar gelagertes, einen Kanal 7a bildendes Stellelement 8a auf, wobei sich der Kanal 7a von einer Stellelement-Einlassöffnung 9a zu einer Stellelement-Auslassöffnung 10a durch das Stellelement 8a hindurch erstreckt. Das Gehäuse 5a der Koppeleinrichtung 4a ist mit dem Gehäuse 5b einer weiteren Koppeleinrichtung 4b mechanisch verbindbar, wobei hinsichtlich der Ausgestaltung der weiteren Koppeleinrichtung 4b auf die Ausführungen zu der vorschlagsgemäßen ersten Koppeleinrichtung 4a verwiesen werden darf. Das Gehäuse 5a weist einen Fluideinlass 11a und einen Fluidauslass 12a auf, die den Gehäuseinnenraum 6a mit der Umgebung verbinden, wobei im mechanisch verbunden Zustand der Gehäuse 5a, 5b der Fluidauslass 12a des Gehäuses 5a der vorschlagsgemäßen Koppeleinrichtung 4a und der Fluideinlass 11b des Gehäuses 5b der weiteren Koppeleinrichtung 4b miteinander in Überdeckung bringbar sind. Im mechanisch miteinander verbundenen Zustand der Gehäuse 5a, 5b ist das Stellelement 8a von einer Ausgangsstellung, in der eine Fluidverbindung zwischen Kanal 7a und Stellelement-Einlassöffnung 9a einerseits und zwischen Kanal 7a und Stellelement-Auslassöffnung 10a andererseits blockiert ist, in eine Betriebsstellung, in der die Stellelement-Einlassöffnung 9a mit dem Fluideinlass 11a und die Stellelement-Auslassöffnung 10a mit dem Fluidauslass 12a in Fluidverbindung steht, verstellbar.

Wesentlich ist nun, dass sich in der Ausgangsstellung der jeweilige Kanal 7a, 7b in einem gegenüber der Umgebung des Gehäuses 5a, 5b hermetisch abgedichteten Bereich des Gehäuseinnenraums 6a, 6b erstreckt.

Auf alle Ausführungen zu dem vorschlagsgemäßen Sterilverbinder 1 darf insoweit verwiesen werden.

Nach einer weiteren Lehre, der ebenfalls eigenständige Bedeutung zukommt, wird eine Verpackungsanordnung 19, 21 mit einer Verpackung 20, 22 und mit mindestens einer, vorzugsweise genau einer, darin steril verpackten, vor schlagsgemäßen Koppeleinrichtung 4a, 4b oder einem darin steril verpackten, vorschlagsgemäßen Sterilverbinder 1 beansprucht. Auf alle Ausführungen zu dem vorschlagsgemäßen Sterilverbinder 1 und der vorschlagsgemäßen Koppeleinrichtung 4a darf insoweit verwiesen werden.

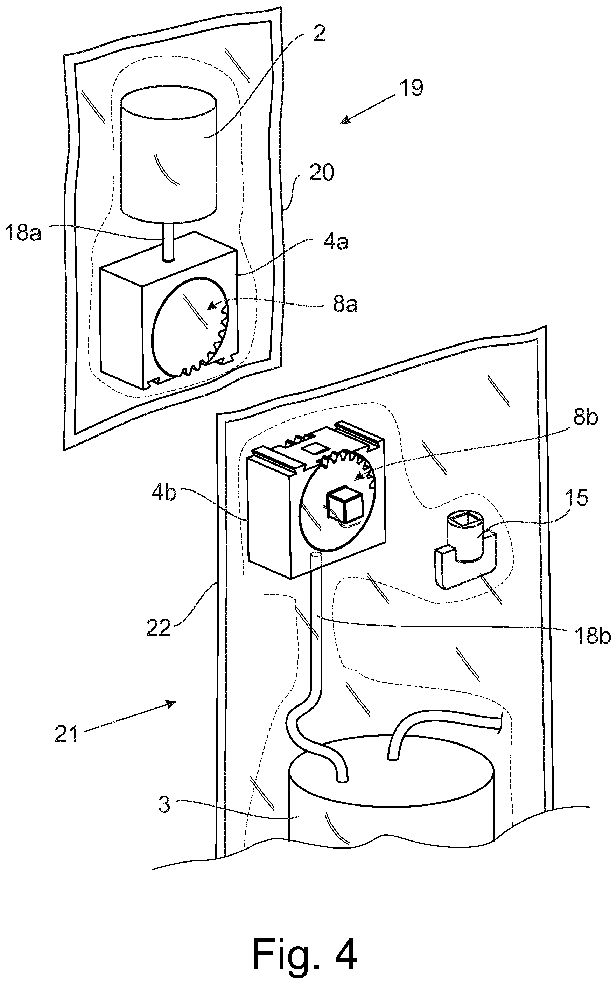

Gemäß einem Ausführungsbeispiel ist eine Verpackungsanordnung 19, wie sie in Fig. 4 oben dargestellt ist, mit einer Verpackung 20 und einer darin steril verpackten, vorschlagsgemäßen Koppeleinrichtung 4a sowie einem darin steril verpackten, insbesondere gefüllten, hier bereits herstellerseitig befüllten, Flüssigkeitsbehälter 2 vorgesehen. Der Flüssigkeitsbehälter 2 ist dabei mit der vorschlagsgemäßen Koppeleinrichtung 4a des Sterilverbinders 1, also schon im verpackten Zustand, fluidtechnisch gekoppelt und das Steuerelement 8a der Koppeleinrichtung 4a ist dabei in seiner Ausgangstellung.

Gemäß einem weiteren Ausführungsbeispiel ist eine Verpackungsanordnung 21, wie sie in Fig. 4 unten dargestellt ist, mit einer Verpackung 22 und einer darin steril verpackten, vorschlagsgemäßen Koppeleinrichtung 4b sowie einer darin steril verpackten bioprozesstechnischen Anlage 3, hier einem Bioreaktor, vorgesehen. Die bioprozesstechnische Anlage 3 bzw. der Bioreaktor ist dabei mit der vorschlagsgemäßen Koppeleinrichtung 4b des Sterilverbinders 1, also im verpackten Zustand, fluidtechnisch gekoppelt und das Stellelement 8b der Koppeleinrichtung 4b ist in seiner Ausgangsstellung. Hier und vorzugsweise weist die Verpackungsanordnung 21 zusätzlich auch ein Betätigungselement 15 auf, das später im zusammengesetzten Zustand des Sterilverbinders 1, wenn also wie beschrieben die Gehäuse 5a, 5b miteinander verbunden sind, zur manuellen Betätigung des Getriebes 14 und entsprechend zur Verstellung der Stellelemente 8a, 8b von der Ausgangsstellung in die Betriebsstellung dient.

In einem alternativen, hier nicht dargestellten Ausführungsbeispiel ist auch eine Verpackungsanordnung mit einer Verpackung denkbar, in der allein ein vorschlagsgemäßer Sterilverbinder 1, umfassend die beiden Koppeleinrichtungen 4a, 4b, steril verpackt ist. Grundsätzlich ist es auch denkbar, eine Verpackungsanordnung bereit zu stellen, in der allein die jeweilige Koppeleinrichtung 4a, 4b steril verpackt ist.

Nach noch einer weiteren Lehre, der ebenfalls eigenständige Bedeutung zukommt, wird eine Verwendung eines steril verpackten, vorschlagsgemäßen Sterilverbinders 1, einer steril verpackten vorschlagsgemäßen Koppeleinrichtung 4a, 4b und/oder einer vorschlagsgemäßen Verpackungsanordnung 19, 21 für den sterilen Transfer eines flüssigen, insbesondere biologischen, Mediums von einem Flüssigkeitsbehälter 2 in eine bioprozesstechnische Anlage 3, insbesondere in einen Bioreaktor, als solche beansprucht. Auch hinsichtlich der beanspruchten Verwendung darf insoweit auf alle Ausführungen zu dem vorschlagsgemäßen Sterilverbinder 1, der vorschlagsgemäßen Koppeleinrichtung 4a, 4b und der vorschlagsgemäßen Verpackungsanordnung 19, 21 verwiesen werden.

Gemäß dem in Fig. 4 gezeigten Ausführungsbeispiel wird nach dem Entpacken der Einheit aus Koppeleinrichtung 4a und Flüssigkeitsbehälter 2 einerseits und nach dem Entpacken der Einheit aus Koppeleinrichtung 4b und bioprozesstechnischer Anlage 3 andererseits, jeweils mit den die Verbindung herstellenden Schläuchen 18a, 18b oder Röhrchen, die eine Koppeleinrichtung 4a mit der anderen Koppeleinrichtung 4b wie beschrieben verbunden. Anschließend werden die Stellelemente 8a, 8b, insbesondere synchron, von ihrer Ausgangsstellung in ihre Betriebsstellung verstellt, sodass dann der Transfer des flüssigen Mediums vom Flüssigkeitsbehälter 2 über die Koppeleinrichtung 4a und die Koppeleinrichtung 4b in die bioprozesstechnische Anlage 3 erfolgen kann.

In besonders bevorzugter Ausgestaltung handelt es sich zumindest bei der vorschlagsgemäßen Koppeleinrichtung 4a oder der vorschlagsgemäßen Koppeleinrichtung 4b, insbesondere bei beiden Koppeleinrichtungen 4a, 4b, vorzugsweise auch bei dem Flüssigkeitsbehälter 2 und/oder bei der bioprozesstechnischen Anlage 3 und/oder bei dem oder den Schläuchen 18a, 18b oder Röhrchen, jeweils um eine Singe-Use-Komponente. Die jeweilige Komponente, das heißt die jeweilige Koppeleinrichtung 4a, 4b, der Flüssigkeitsbehälter 2, die bioprozesstechnische Anlage 3 und/oder der jeweilige Schlauch 18a, 18b bzw. das jeweilige Röhrchen, ist zumindest zum Teil, vorzugsweise zumindest überwiegend, aus einem Kunststoffmaterial ausgelegt. Als Kunststoffe für die einzelnen Komponenten kommen insbesondere ein Silikonmaterial und/oder ein Polymermaterial, insbesondere ein Elastomer und/oder Thermoplast und/oder Vero-Material, in Frage. Beispiele hierfür sind PE (Polyethylen), PP (Polypropylen), PTFE (Polytetrafluorethylen), PBT (Polybutylenterephthalat), PSU (Polysulfon), PESU (Polyethersulfon), PC (Polycarbonat).

## Patentansprüche

1. Sterilverbinder für den sterilen Transfer eines flüssigen, insbesondere biologischen, Mediums von einem Flüssigkeitsbehälter (2) in eine bioprozesstechnische Anlage (3), insbesondere in einen Bioreaktor,
wobei der Sterilverbinder (1) zwei Koppeleinrichtungen (4a, 4b) aufweist,
wobei die Koppeleinrichtungen (4a, 4b) jeweils ein Gehäuse (5a, 5b) mit einem Gehäuseinnenraum (6a, 6b) und ein im Gehäuseinnenraum (6a, 6b) verstellbar gelagertes, einen Kanal (7a, 7b) bildendes Stellelement (8a, 8b) aufweisen, wobei sich der Kanal (7a, 7b) von einer Stellelement-Einlassöffnung (9a, 9b) zu einer Stellelement-Auslassöffnung (10a, 10b) durch das Stellelement (8a, 8b) hindurch erstreckt,
wobei das Gehäuse (5a) der einen Koppeleinrichtung (4a) mit dem Gehäuse (5b) der anderen Koppeleinrichtung (4b) mechanisch verbindbar ist,
wobei das jeweilige Gehäuse (5a, 5b) einen Fluideinlass (11a, 11b) und einen Fluidauslass (12a, 12b) aufweist, die den Gehäuseinnenraum (6a, 6b) mit der Umgebung verbinden, wobei im mechanisch miteinander verbundenen Zustand der Gehäuse (5a, 5b) der Fluidauslass (12a) des einen Gehäuses (5a) und der Fluideinlass (11b) des anderen Gehäuses (5b) miteinander in Überdeckung sind,
wobei im mechanisch miteinander verbundenen Zustand der Gehäuse (5a, 5b) die Stellelemente (8a, 8b) jeweils von einer Ausgangsstellung, in der eine Fluidverbindung zwischen Kanal (7a, 7b) und Stellelement-Einlassöffnung (9a, 9b) einerseits und zwischen Kanal (7a, 7b) und Stellelement-Auslassöffnung (10a, 10b) andererseits blockiert ist, in eine Betriebsstellung, in der die Stellelement-Einlassöffnung (9a, 9b) mit dem Fluideinlass (11a, 11b) und die Stellelement-Auslassöffnung (10a, 10b) mit dem Fluidauslass (12a, 12b) in Fluidverbindung steht, verstellbar sind,
**dadurch gekennzeichnet,**
**dass** sich in der Ausgangsstellung der jeweilige Kanal (7a, 7b) in einem gegenüber der Umgebung des Gehäuses (5a, 5b) hermetisch abgedichteten Bereich des Gehäuseinnenraums (6a, 6b) erstreckt.

2. Sterilverbinder nach Anspruch 1, **dadurch gekennzeichnet, dass** in der Ausgangsstellung die Stellelement-Einlassöffnung (9a, 9b) und die Stellelement-Auslassöffnung (10a, 10b) jeweils zu der Gehäuseinnenwand (13a, 13b) des jeweiligen Gehäuses (5a, 5b) weisen.

3. Sterilverbinder nach Anspruch 1 oder 2, **dadurch gekennzeichnet, dass** in der Ausgangsstellung ein Abschnitt, insbesondere ein Umfangsabschnitt (Ua₁, Ub₁), des jeweiligen Stellelements (8a, 8b) den Fluideinlass (11a, 11b) und/oder ein Abschnitt, insbesondere ein Umfangsabschnitt (Ua₂, Ub₂), des jeweiligen Stellelements (8a, 8b) den Fluidauslass (12a, 12b) des jeweiligen Gehäuses (5a, 5b) hermetisch dicht verschließt, und/oder,
dass in der Ausgangsstellung ein um die Stellelement-Einlassöffnung (9a, 9b) herum verlaufender Abschnitt, insbesondere Umfangsabschnitt (Ua₃, Ub₃), des jeweiligen Stellelements (8a, 8b) und/oder ein um die Stellelement-Auslassöffnung (10a, 10b) herum verlaufender Abschnitt, insbesondere Umfangsabschnitt (Ua₄, Ub₄), des jeweiligen Stellelements (8a, 8b) hermetisch dichtend an der Gehäuseinnenwand (13a, 13b) des jeweiligen Gehäuses (5a, 5b) anliegt, und/oder,
dass in der Ausgangsstellung ein um den Fluideinlass (11a, 11b) herum verlaufender Abschnitt (Aa₁, Ab₁) des jeweiligen Gehäuses (5a, 5b) und/oder ein um den Fluidauslass (12a, 12b) herum verlaufender Abschnitt (Aa₂, Ab₂) des jeweiligen Gehäuses (5a, 5b) hermetisch dichtend an dem jeweiligen Stellelement (8a, 8b) anliegt.

4. Sterilverbinder nach einem der vorhergehenden Ansprüche, **dadurch gekennzeichnet, dass** das Stellelement (8a) des einen Gehäuses (5a) durch den Fluidauslass (12a) des Gehäuses (5a) aus dem Gehäuseinnenraum (6a) herausragt, und/oder, dass das Stellelement (8b) des anderen Gehäuses (5b) durch den Fluideinlass (11b) des Gehäuses (5b) aus dem Gehäuseinnenraum (6b) herausragt.

5. Sterilverbinder nach einem der vorhergehenden Ansprüche, **dadurch gekennzeichnet, dass** das Stellelement (8a) des einen Gehäuses (5a) und das Stellelement (8b) des anderen Gehäuses (5b) im mechanisch miteinander verbundenen Zustand der Gehäuse (5a, 5b) zumindest dann, wenn beide Stellelemente (8a, 8b) in ihrer Betriebsstellung sind, vorzugsweise auch dann, wenn beide Stellelemente (8a, 8b) in ihrer Ausgangsstellung sind, weiter vorzugsweise ständig, unter Vorspannung aneinander anliegen, vorzugsweise, dass sich im Bereich des gegenseitigen Kontakts die Stellelemente (8a, 8b) elastisch verformen.

6. Sterilverbinder nach einem der vorhergehenden Ansprüche, **dadurch gekennzeichnet, dass** im mechanisch miteinander verbundenen Zustand der Gehäuse (5a, 5b) dann, wenn beide Stellelemente (8a, 8b) in ihrer Betriebsstellung sind, ein um die Stellelement-Auslassöffnung (10a) herum verlaufender Abschnitt, insbesondere Umfangsabschnitt (Ua₄), des einen Stellelements (8a) und ein um die Stellelement-Einlassöffnung (9b) herum verlaufender Abschnitt, insbesondere Umfangsabschnitt (Ub₃), des anderen Stellelements (8b), den jeweiligen Kanal (7a, 7b) zum jeweiligen Gehäuse (5a, 5b) hin hermetisch abdichtend, aneinander anliegen.

7. Sterilverbinder nach einem der vorhergehenden Ansprüche, **dadurch gekennzeichnet, dass** das jeweilige Stellelement (8a, 8b) abschnittsweise oder vollständig, insbesondere in dem Abschnitt, der den Fluideinlass (11a, 11b) hermetisch dicht verschließt, und/oder in dem Abschnitt, der den Fluidauslass (12a, 12b) hermetisch dicht verschließt, und/oder in dem um die Stellelement-Einlassöffnung (9a, 9b) herum verlaufenden Abschnitt und/oder in dem um die Stellelement-Auslassöffnung (10a, 10b) herum verlaufenden Abschnitt, aus einem Elastomer, insbesondere aus einem Silikon-Material, ausgelegt ist, und/oder,
dass das jeweilige Gehäuse (5a, 5b) abschnittsweise, insbesondere in dem um den Fluideinlass (11a, 11b) herum verlaufenden Abschnitt und/oder in dem um den Fluidauslass (12a, 12b) herum verlaufenden Abschnitt, aus einem Elastomer, insbesondere aus einem Silikon-Material, und vorzugsweise im Übrigen aus einem Kunststoff mit einer geringeren Elastizität, insbesondere einem Thermoplast, ausgelegt ist.

8. Sterilverbinder nach einem der vorhergehenden Ansprüche, **dadurch gekennzeichnet, dass** im mechanisch miteinander verbundenen Zustand der Gehäuse (5a, 5b), wenn beide Stellelemente (8a, 8b) in ihrer Betriebsstellung sind, bei beiden Koppeleinrichtungen (4a, 4b) jeweils Fluideinlass (11a, 11b) und Stellelement-Einlassöffnung (9a, 9b) und/oder Fluidauslass (12a, 12b) und Stellelement-Auslassöffnung (10a, 10b) miteinander in Überdeckung sind, insbesondere koaxial zueinander ausgerichtet, und der Fluidauslass (12a) des einen Gehäuses (5a) und der Fluideinlass (11b) des anderen Gehäuses (5b) miteinander in Überdeckung sind, insbesondere koaxial zueinander ausgerichtet sind, vorzugsweise, dass im mechanisch miteinander verbundenen Zustand der Gehäuse (5a, 5b), wenn beide Stellelemente (8a, 8b) in ihrer Betriebsstellung sind, die beiden Fluideinlässe (11a, 11b), die beiden Fluidauslässe (12a, 12b) und die beiden Kanäle (7a, 7b) koaxial zueinander ausgerichtet sind.

9. Sterilverbinder nach einem der vorhergehenden Ansprüche, **dadurch gekennzeichnet, dass** im mechanisch miteinander verbundenen Zustand der Gehäuse (5a, 5b) eine Verstellbewegung des einen Stellelements (8a, 8b) zwischen seiner Ausgangsstellung und seiner Betriebsstellung mit einer korrespondierenden Verstellbewegung des anderen Stellelements (8b, 8a) einhergeht, vorzugsweise, dass ein Getriebe (14), insbesondere Zahnradgetriebe, vorgesehen ist, das im mechanisch miteinander verbundenen Zustand der Gehäuse (5a, 5b) ein synchrones Verstellen der beiden Stellelemente (8a, 8b) zwischen ihre Ausgangsstellung und ihre Betriebsstellung bewirkt, weiter vorzugsweise, dass eine Getriebekomponente des Getriebes (14), insbesondere ein Zahnrad oder Zahnradsegment des Getriebes (14), mit einem Betätigungselement (15) drehfest verbunden oder verbindbar ist.

10. Sterilverbinder nach einem der vorhergehenden Ansprüche, **dadurch gekennzeichnet, dass** die Verstellbewegung des jeweiligen Stellelements (8a, 8b) zwischen seiner Ausgangsstellung und seiner Betriebsstellung eine rotatorische Bewegung oder eine lineare Bewegung ist.

11. Sterilverbinder nach einem der vorhergehenden Ansprüche, **dadurch gekennzeichnet, dass** die Verstellbewegung des jeweiligen Stellelements (8a, 8b) zwischen seiner Ausgangsstellung und seiner Betriebsstellung in der Ausgangsstellung und/oder Betriebsstellung über einen Anschlag (16a, 16b, 17a, 17b) begrenzt ist.

12. Sterilverbinder nach einem der vorhergehenden Ansprüche, **dadurch gekennzeichnet, dass** der Fluideinlass (11a) des einen Gehäuses (5a) und der Fluidauslass (12b) des anderen Gehäuses (5b), insbesondere über eine Steckverbindung, mit einem Schlauch (18a, 18b) oder Röhrchen verbindbar oder verbunden ist, vorzugsweise, dass der Fluideinlass (11a) des einen Gehäuses (5a) über den Schlauch (18a) oder das Röhrchen mit einem Flüssigkeitsbehälter (2), insbesondere mit einem Mindermengen-Flüssigkeitsbehälter, zur Abgabe eines flüssigen, insbesondere biologischen, Mediums fluidtechnisch gekoppelt ist, und/oder, dass der Fluidauslass (12b) des anderen Gehäuses (5b) über den Schlauch (18b) oder das Röhrchen mit einer bioprozesstechnischen Anlage (3), insbesondere einem Bioreaktor, fluidtechnisch gekoppelt ist.

13. Sterilverbinder nach einem der vorhergehenden Ansprüche, **dadurch gekennzeichnet, dass** die beiden Gehäuse (5a, 5b) formschlüssig miteinander verbindbar, insbesondere miteinander zusammensteckbar oder verrastbar, sind.

14. Koppeleinrichtung eines Sterilverbinders (1) für den sterilen Transfer eines flüssigen, insbesondere biologischen, Mediums von einem Flüssigkeitsbehälter (2) in eine bioprozesstechnische Anlage (3), insbesondere in einen Bioreaktor, insbesondere Sterilverbinder (1) nach einem der vorhergehenden Ansprüche, wobei die Koppeleinrichtung (4a, 4b) ein Gehäuse (5a, 5b) mit einem Gehäuseinnenraum (6a, 6b) und ein im Gehäuseinnenraum (6a, 6b) verstellbar gelagertes, einen Kanal (7a, 7b) bildendes Stellelement (8a, 8b) aufweist, wobei sich der Kanal (7a, 7b) von einer Stellelement-Einlassöffnung (9a, 9b) zu einer Stellelement-Auslassöffnung (10a, 10b) durch das Stellelement (8a, 8b) hindurch erstreckt,
wobei das Gehäuse (5a, 5b) der Koppeleinrichtung (4a, 4b) mit dem Gehäuse (5b, 5a) einer weiteren Koppeleinrichtung (4b, 4a) des Sterilverbinders (1) mechanisch verbindbar ist,
wobei das Gehäuse (5a, 5b) einen Fluideinlass (11a, 11b) und einen Fluidauslass (12a, 12b) aufweist, die den Gehäuseinnenraum (6a, 6b) mit der Umgebung verbinden, wobei im mechanisch verbunden Zustand der Gehäuse (5a, 5b) der Fluidauslass (12a, 12b) des Gehäuses (5a, 5b) der Koppeleinrichtung (4a, 4b) und der Fluideinlass (11b, 11a) des Gehäuses (5b, 5a) der weiteren Koppeleinrichtung (4b, 4a) miteinander in Überdeckung bringbar sind,
wobei im mechanisch miteinander verbundenen Zustand der Gehäuse (5a, 5b) das Stellelement (8a, 8b) von einer Ausgangsstellung, in der eine Fluidverbindung zwischen Kanal (7a, 7b) und Stellelement-Einlassöffnung (9a, 9b) einerseits und zwischen Kanal (7a, 7b) und Stellelement-Auslassöffnung (10a, 10b) andererseits blockiert ist, in eine Betriebsstellung, in der die Stellelement-Einlassöffnung (9a, 9b) mit dem Fluideinlass (11a, 11b) und die Stellelement-Auslassöffnung (10a, 10b) mit dem Fluidauslass (12a, 12b) in Fluidverbindung steht, verstellbar ist,
**dadurch gekennzeichnet,**
**dass** sich in der Ausgangsstellung der jeweilige Kanal (7a, 7b) in einem gegenüber der Umgebung des Gehäuses (5a, 5b) hermetisch abgedichteten Bereich des Gehäuseinnenraums (6a, 6b) erstreckt.

15. Verpackungsanordnung mit einer Verpackung (20, 22) und mit mindestens einer, vorzugsweise genau einer, darin steril verpackten Koppeleinrichtung (4a, 4b) nach Anspruch 14 oder einem darin steril verpackten Sterilverbinder (1) nach einem der Ansprüche 1 bis 13.

16. Verpackungsanordnung nach Anspruch 15, **dadurch gekennzeichnet, dass** das Stellelement (8a, 8b) der in der Verpackung (20, 22) steril verpackten Koppeleinrichtung (4a, 4b) in seiner Ausgangsstellung ist und dass in der Verpackung (20, 22) außerdem ein, insbesondere gefüllter, Flüssigkeitsbehälter (2) steril verpackt ist, der mit der Koppeleinrichtung (4a) fluidtechnisch gekoppelt ist, oder eine bioprozesstechnische Anlage (3) steril verpackt ist, die mit der Koppeleinrichtung (4b) fluidtechnisch gekoppelt ist.

17. Verwendung eines steril verpackten Sterilverbinders (1) nach einem der Ansprüche 1 bis 13, einer steril verpackten Koppeleinrichtung (4a, 4b) nach Anspruch 14 und/oder einer Verpackungsanordnung (19, 21) nach Anspruch 15 oder 16 für den sterilen Transfer eines flüssigen, insbesondere biologischen, Mediums von einem Flüssigkeitsbehälter (2) in eine bioprozesstechnische Anlage (3), insbesondere in einen Bioreaktor.

18. Verwendung nach Anspruch 17, **dadurch gekennzeichnet, dass** zumindest eine Koppeleinrichtung, insbesondere beide Koppeleinrichtungen (4a, 4b), vorzugsweise auch der Flüssigkeitsbehälter (2) und/oder die bioprozesstechnische Anlage (3) und/oder der oder die Schläuche (18a, 18b) oder Röhrchen, insbesondere als Single-Use-Komponente, zumindest zum Teil, vorzugsweise zumindest überwiegend, aus einem Kunststoffmaterial ausgelegt sind.

## Claims

1. Sterile connector for the sterile transfer of a liquid medium, in particular a biological medium, from a liquid container (2) to a bioprocess engineering system (3), in particular to a bioreactor,
wherein the sterile connector (1) has two coupling devices (4a, 4b),
wherein the coupling devices (4a, 4b) each have a housing (5a, 5b) having a housing interior (6a, 6b) and a control element (8a, 8b) which is adjustably mounted in the housing interior (6a, 6b) and forms a channel (7a, 7b), wherein the channel (7a, 7b) extends through the control element (8a, 8b) from a control element inlet opening (9a, 9b) to a control element outlet opening (10a, 10b),
wherein the housing (5a) of one coupling device (4a) is able to be mechanically connected to the housing (5b) of the other coupling device (4b),
wherein each housing (5a, 5b) has a fluid inlet (11a, 11b) and a fluid outlet (12a, 12b) which connect the housing interior (6a, 6b) to the surroundings, wherein, in the mechanically connected state of the housings (5a, 5b), the fluid outlet (12a) of one housing (5a) and the fluid inlet (11b) of the other housing (5b) overlap,
wherein, in the mechanically connected state of the housings (5a, 5b), the control elements (8a, 8b) are each adjustable from a starting position, in which a fluid connection between the channel (7a, 7b) and the control element inlet opening (9a, 9b) on the one hand and between the channel (7a, 7b) and the control element outlet opening (10a, 10b) on the other hand is blocked, into an operating position, in which the control element inlet opening (9a, 9b) is fluidically connected to the fluid inlet (11a, 11b) and the control element outlet opening (10a, 10b) is fluidically connected to the fluid outlet (12a, 12b),
**characterized in that**,
in the starting position, each channel (7a, 7b) extends in a region of the housing interior (6a, 6b) that is hermetically sealed with respect to the surroundings of the housing (5a, 5b).

2. Sterile connector according to claim 1, **characterized in that**, in the starting position, the control element inlet opening (9a, 9b) and the control element outlet opening (10a, 10b) each face the housing inner wall (13a, 13b) of the respective housing (5a, 5b).

3. Sterile connector according to claim 1 or 2, **characterized in that**, in the starting position, a portion, in particular a circumferential portion (Ua₁, Ub₁), of the respective control element (8a, 8b) closes the fluid inlet (11a, 11b) and/or a portion, in particular a circumferential portion (Ua₂, Ub₂), of the respective control element (8a, 8b) closes the fluid outlet (12a, 12b) of the respective housing (5a, 5b) in a hermetically tight manner, and/or
**in that**, in the starting position, a portion, in particular a circumferential portion (Ua₃, Ub₃), of the respective control element (8a, 8b) extending around the control element inlet opening (9a, 9b) and/or a portion, in particular a circumferential portion (Ua₄, Ub₄), of the respective control element (8a, 8b) extending around the control element outlet opening (10a, 10b) is in contact in a hermetically sealing manner with the housing inner wall (13a, 13b) of the respective housing (5a, 5b), and/or
**in that**, in the starting position, a portion (Aa₁, Ab₁) of the respective housing (5a, 5b) extending around the fluid inlet (11a, 11b) and/or a portion (Aa₂, Ab₂) of the respective housing (5a, 5b) extending around the fluid outlet (12a, 12b) is in contact in a hermetically sealing manner with the respective control element (8a, 8b).

4. Sterile connector according to any one of the preceding claims, **characterized in that** the control element (8a) of one housing (5a) projects from the housing interior (6a) through the fluid outlet (12a) of the housing (5a), and/or **in that** the control element (8b) of the other housing (5b) projects from the housing interior (6b) through the fluid inlet (11b) of the housing (5b).

5. Sterile connector according to any one of the preceding claims, **characterized in that** the control element (8a) of one housing (5a) and the control element (8b) of the other housing (5b), in the mechanically connected state of the housings (5a, 5b), at least when the two control elements (8a, 8b) are in their operating position, preferably also when the two control elements (8a, 8b) are in their starting position, more preferably always, are in contact with one another under preload, preferably **in that** the contact elements (8a, 8b) deform elastically in the region of mutual contact.

6. Sterile connector according to any one of the preceding claims, **characterized in that**, in the mechanically connected state of the housings (5a, 5b), when the two control elements (8a, 8b) are in their operating position, a portion, in particular a circumferential portion (Ua₄), of one control element (8a) extending around the control element outlet opening (10a) and a portion, in particular a circumferential portion (Ub₃), of the other control element (8b) extending around the control element inlet opening (9b) are in contact with one another in such a manner that the respective channel (7a, 7b) is hermetically sealed with respect to the respective housing (5a, 5b).

7. Sterile connector according to any one of the preceding claims, **characterized in that** each control element (8a, 8b) is produced in part or completely, in particular in the portion that closes the fluid inlet (11a, 11b) in a hermetically tight manner, and/or in the portion that closes the fluid outlet (12a, 12b) in a hermetically tight manner, and/or in the portion extending around the control element inlet opening (9a, 9b) and/or in the portion extending around the control element outlet opening (10a, 10b), from an elastomer, in particular from a silicone material, and/or
**in that** each housing (5a, 5b) is produced in part, in particular in the portion extending around the fluid inlet (11a, 11b) and/or in the portion extending around the fluid outlet (12a, 12b), from an elastomer, in particular from a silicone material, and preferably for the rest from a plastics material with relatively low elasticity, in particular from a thermoplastic.

8. Sterile connector according to any one of the preceding claims, **characterized in that**, in the mechanically connected state of the housings (5a, 5b), when the two control elements (8a, 8b) are in their operating position, in the case of both coupling devices (4a, 4b) in each case the fluid inlet (11a, 11b) and the control element inlet opening (9a, 9b) and/or the fluid outlet (12a, 12b) and the control element outlet opening (10a, 10b) overlap, in particular are oriented coaxially with one another, and the fluid outlet (12a) of one housing (5a) and the fluid inlet (11b) of the other housing (5b) overlap, in particular are oriented coaxially with one another, preferably in that, in the mechanically connected state of the housings (5a, 5b), when the two control elements (8a, 8b) are in their operating position, the two fluid inlets (11a, 11b), the two fluid outlets (12a, 12b) and the two channels (7a, 7b) are oriented coaxially with one another.

9. Sterile connector according to any one of the preceding claims, **characterized in that**, in the mechanically connected state of the housings (5a, 5b), an adjusting movement of one control element (8a, 8b) between its starting position and its operating position is accompanied by a corresponding adjusting movement of the other control element (8b, 8a), preferably **in that** there is provided a transmission (14), in particular a gear train, which, in the mechanically connected state of the housings (5a, 5b), effects a synchronous adjustment of the two control elements (8a, 8b) between their starting position and their operating position, more preferably **in that** a transmission component of the transmission (14), in particular a gear wheel or gear wheel segment of the transmission (14), is connected or is able to be connected in a rotationally fixed manner to an actuating element (15).

10. Sterile connector according to any one of the preceding claims, **characterized in that** the adjusting movement of each control element (8a, 8b) between its starting position and its operating position is a rotational movement or a linear movement.

11. Sterile connector according to any one of the preceding claims, **characterized in that** the adjusting movement of each control element (8a, 8b) between its starting position and its operating position is limited in the starting position and/or operating position via a stop (16a, 16b, 17a, 17b).

12. Sterile connector according to any one of the preceding claims, **characterized in that** the fluid inlet (11a) of one housing (5a) and the fluid outlet (12b) of the other housing (5b) is able to be connected or is connected, in particular via a plug-in connection, to a flexible tube (18a, 18b) or a pipe, preferably **in that** the fluid inlet (11a) of one housing (5a) is fluidically coupled via the flexible tube (18a) or the pipe with a liquid container (2), in particular with a minimum-quantity liquid container, for the delivery of a liquid medium, in particular a biological medium, and/or **in that** the fluid outlet (12b) of the other housing (5b) is fluidically coupled via the flexible tube (18b) or the pipe with a bioprocess engineering system (3), in particular a bioreactor.

13. Sterile connector according to any one of the preceding claims, **characterized in that** the two housings (5a, 5b) are able to be connected together in a form-fitting manner, in particular are able to be pushed together or latched together.

14. Coupling device of a sterile connector (1) for the sterile transfer of a liquid medium, in particular a biological medium, from a liquid container (2) to a bioprocess engineering system (3), in particular to a bioreactor, in particular a sterile connector (1) according to any one of the preceding claims,
wherein the coupling device (4a, 4b) has a housing (5a, 5b) having a housing interior (6a, 6b) and a control element (8a, 8b) which is adjustably mounted in the housing interior (6a, 6b) and forms a channel (7a, 7b), wherein the channel (7a, 7b) extends through the control element (8a, 8b) from a control element inlet opening (9a, 9b) to a control element outlet opening (10a, 10b),
wherein the housing (5a, 5b) of the coupling device (4a, 4b) is able to be mechanically connected to the housing (5b, 5a) of a further coupling device (4b, 4a) of the sterile connector (1),
wherein the housing (5a, 5b) has a fluid inlet (11a, 11b) and a fluid outlet (12a, 12b) which connect the housing interior (6a, 6b) to the surroundings, wherein, in the mechanically connected state of the housings (5a, 5b), the fluid outlet (12a, 12b) of the housing (5a, 5b) of the coupling device (4a, 4b) and the fluid inlet (11b, 11a) of the housing (5b, 5a) of the further coupling device (4b, 4a) are able to be brought to overlap,
wherein, in the mechanically connected state of the housings (5a, 5b), the control element (8a, 8b) is adjustable from a starting position, in which a fluid connection between the channel (7a, 7b) and the control element inlet opening (9a, 9b) on the one hand and between the channel (7a, 7b) and the control element outlet opening (10a, 10b) on the other hand is blocked, into an operating position, in which the control element inlet opening (9a, 9b) is fluidically connected to the fluid inlet (11a, 11b) and the control element outlet opening (10a, 10b) is fluidically connected to the fluid outlet (12a, 12b),
**characterized in that**,
in the starting position, each channel (7a, 7b) extends in a region of the housing interior (6a, 6b) that is hermetically sealed with respect to the surroundings of the housing (5a, 5b).

15. Packaging arrangement having a packaging (20, 22) and having at least one, preferably exactly one, coupling device (4a, 4b) according to claim 14 sterile packaged therein or a sterile connector (1) according to any one of claims 1 to 13 sterile packaged therein.

16. Packaging arrangement according to claim 15, **characterized in that** the control element (8a, 8b) of the coupling device (4a, 4b) sterile packaged in the packaging (20, 22) is in its starting position, and **in that** there is additionally sterile packaged in the packaging (20, 22) a liquid container (2), in particular a filled liquid container, which is fluidically coupled with the coupling device (4a), or there is sterile packaged a bioprocess engineering system (3) which is fluidically coupled with the coupling device (4b).

17. Use of a sterile packaged sterile connector (1) according to any one of claims 1 to 13, of a sterile packaged coupling device (4a, 4b) according to claim 14 and/or of a packaging arrangement (19, 21) according to claim 15 or 16 for the sterile transfer of a liquid medium, in particular a biological medium, from a liquid container (2) to a bioprocess engineering system (3), in particular to a bioreactor.

18. Use according to claim 17, **characterized in that** at least one coupling device, in particular both coupling devices (4a, 4b), preferably also the liquid container (2) and/or the bioprocess engineering system (3) and/or the flexible tube(s) (18a, 18b) or the pipe(s), in particular as a single-use component, are produced at least in part, preferably at least predominantly, from a plastics material.

## Revendications

1. Connecteur stérile pour le transfert stérile d'un milieu liquide, en particulier biologique, à partir d'un récipient de liquide (2) dans une installation de bioprocédé (3), en particulier dans un bioréacteur,
dans lequel le connecteur stérile (1) présente deux dispositifs d'accouplement (4a, 4b),
dans lequel les dispositifs d'accouplement (4a, 4b) présentent respectivement un boîtier (5a, 5b) avec un espace intérieur de boîtier (6a, 6b) et un élément de commande (8a, 8b) monté réglable dans l'espace intérieur de boîtier (6a, 6b), formant un canal (7a, 7b),
dans lequel le canal (7a, 7b) s'étend à partir d'une ouverture d'entrée d'élément de commande (9a, 9b) vers une ouverture de sortie d'élément de commande (10a, 10b) à travers l'élément de commande (8a, 8b),
dans lequel le boîtier (5a) de l'un des dispositifs d'accouplement (4a) peut être relié mécaniquement au boîtier (5b) de l'autre dispositif d'accouplement (4b),
dans lequel le boîtier (5a, 5b) respectif présente une entrée de fluide (11a, 11b) et une sortie de fluide (12a, 12b), qui relient l'espace intérieur de boîtier (6a, 6b) à l'environnement, dans lequel dans l'état des boîtiers (5a, 5b) reliés mécaniquement l'un à l'autre la sortie de fluide (12a) de l'un des boîtiers (5a) et l'entrée de fluide (11b) de l'autre boîtier (5b) coïncident l'une avec l'autre,
dans lequel dans l'état des boîtiers (5a, 5b) reliés mécaniquement l'un à l'autre les éléments de commande (8a, 8b) sont réglables respectivement à partir d'une position initiale, dans laquelle une liaison fluidique entre le canal (7a, 7b) et l'ouverture d'entrée d'élément de commande (9a, 9b) d'une part et entre le canal (7a, 7b) et l'ouverture de sortie d'élément de commande (10a, 10b) d'autre part est bloquée, dans une position de fonctionnement dans laquelle l'ouverture d'entrée d'élément de commande (9a, 9b) est en liaison fluidique avec l'entrée de fluide (11a, 11b) et l'ouverture de sortie d'élément de commande (10a, 10b) avec la sortie de fluide (12a, 12b),
**caractérisé en ce**
**que** dans la position initiale le canal (7a, 7b) respectif s'étend dans une zone de l'espace intérieur de boîtier (6a, 6b) étanchéifiée hermétiquement par rapport à l'environnement du boîtier (5a, 5b).

2. Connecteur stérile selon la revendication 1, **caractérisé en ce que** dans la position initiale l'ouverture d'entrée d'élément de commande (9a, 9b) et l'ouverture de sortie d'élément de commande (10a, 10b) sont respectivement tournées vers la paroi intérieure de boîtier (13a, 13b) du boîtier (5a, 5b) respectif.

3. Connecteur stérile selon la revendication 1 ou 2, **caractérisé en ce que** dans la position initiale une partie, en particulier une partie périphérique (Ua₁, Ub₁), de l'élément de commande (8a, 8b) respectif ferme l'entrée de fluide (11a, 11b) et/ou une partie, en particulier une partie périphérique (Ua₂, Ub₂), de l'élément de commande (8a, 8b) respectif ferme la sortie de fluide (12a, 12b) du boîtier (5a, 5b) respectif de manière hermétiquement étanche, et/ou,
que dans la position initiale une partie s'étendant autour de l'ouverture d'entrée d'élément de commande (9a, 9b), en particulier partie périphérique (Ua₃, Ub₃), de l'élément de commande (8a, 8b) respectif et/ou une partie s'étendant autour de l'ouverture de sortie d'élément de commande (10a, 10b), en particulier partie périphérique (Ua₄, Ub₄), de l'élément de commande (8a, 8b) respectif s'applique de manière hermétiquement étanche sur la paroi intérieure de boîtier (13a, 13b) du boîtier (5a, 5b) respectif, et/ou,
que dans la position initiale une partie (Aa₁, Ab₁) du boîtier (5a, 5b) respectif s'étendant autour de l'entrée de fluide (11a, 11b) et/ou une partie (Aa₂, Ab₂) du boîtier (5a, 5b) respectif s'étendant autour de la sortie de fluide (12a, 12b) s'applique de manière hermétiquement étanche sur l'élément de commande (8a, 8b) respectif.

4. Connecteur stérile selon l'une quelconque des revendications précédentes, **caractérisé en ce que** l'élément de commande (8a) d'un des boîtiers (5a) fait saillie de l'espace intérieur de boîtier (6a) par la sortie de fluide (12a) du boîtier (5a), et/ou que l'élément de commande (8b) de l'autre boîtier (5b) fait saillie de l'espace intérieur de boîtier (6b) par l'entrée de fluide (11b) du boîtier (5b).

5. Connecteur stérile selon l'une quelconque des revendications précédentes, **caractérisé en ce que** l'élément de commande (8a) d'un des boîtiers (5a) et l'élément de commande (8b) de l'autre boîtier (5b) dans l'état des boîtiers (5a, 5b) reliés mécaniquement l'un à l'autre s'appliquent l'un sur l'autre sous précontrainte au moins lorsque les deux éléments de commande (8a, 8b) sont dans leur position de fonctionnement, de préférence également lorsque les deux éléments de commande (8a, 8b) sont dans leur position initiale, mieux encore en permanence, de préférence, que les éléments de commande (8a, 8b) se déforment élastiquement dans la zone du contact mutuel.

6. Connecteur stérile selon l'une quelconque des revendications précédentes, **caractérisé en ce que** dans l'état des boîtiers (5a, 5b) reliés mécaniquement l'un à l'autre, lorsque les deux éléments de commande (8a, 8b) sont dans leur position de fonctionnement, une partie, en particulier partie périphérique (Ua₄), de l'un des éléments de commande (8a) s'étendant autour de l'ouverture de sortie d'élément de commande (10a), et une partie, en particulier partie périphérique (Ub₃), de l'autre élément de commande (8b) s'étendant autour de l'ouverture d'entrée d'élément de commande (9b), s'appliquent l'une sur l'autre de manière à étanchéifier hermétiquement le boîtier (5a, 5b) respectif par rapport au canal (7a, 7b) respectif.

7. Connecteur stérile selon l'une quelconque des revendications précédentes, **caractérisé en ce que** l'élément de commande (8a, 8b) respectif sur certaines parties ou entièrement, en particulier dans la partie qui ferme l'entrée de fluide (11a, 11b) de manière hermétiquement étanche, et/ou dans la partie qui ferme la sortie de fluide (12a, 12b) de manière hermétiquement étanche, et/ou dans la partie s'étendant autour de l'ouverture d'entrée d'élément de commande (9a, 9b) et/ou dans la partie s'étendant autour de l'ouverture de sortie d'élément de commande (10a, 10b), est conçu à partir d'un élastomère, en particulier à partir d'un matériau silicone, et/ou,
que le boîtier (5a, 5b) respectif sur certaines parties, en particulier dans la partie s'étendant autour de l'entrée de fluide (11a, 11b) et/ou dans la partie s'étendant autour de la sortie de fluide (12a, 12b), est conçu à partir d'un élastomère, en particulier à partir d'un matériau silicone, et de préférence est conçu du reste à partir d'une matière plastique avec une élasticité moindre, en particulier un thermoplaste.

8. Connecteur stérile selon l'une quelconque des revendications précédentes, **caractérisé en ce que** dans l'état des boîtiers (5a, 5b) reliés mécaniquement l'un à l'autre, lorsque les deux éléments de commande (8a, 8b) sont dans leur position de fonctionnement, pour les deux dispositifs d'accouplement (4a, 4b) respectivement l'entrée de fluide (11a, 11b) et l'ouverture d'entrée d'élément de commande (9a, 9b) et/ou la sortie de fluide (12a, 12b) et l'ouverture de sortie d'élément de commande (10a, 10b) coïncident l'une avec l'autre, en particulier orientées coaxialement l'une par rapport à l'autre, et la sortie de fluide (12a) d'un des boîtiers (5a) et l'entrée de fluide (11b) de l'autre boîtier (5b) coïncident l'une avec l'autre, en particulier sont orientées coaxialement l'une par rapport à l'autre, de préférence, que dans l'état des boîtiers (5a, 5b) reliés mécaniquement l'un à l'autre, lorsque les deux éléments de commande (8a, 8b) sont dans leur position de fonctionnement, les deux entrées de fluide (11a, 11b), les deux sorties de fluide (12a, 12b) et les deux canaux (7a, 7b) sont orientés coaxialement les uns par rapport aux autres.

9. Connecteur stérile selon l'une quelconque des revendications précédentes, **caractérisé en ce que** dans l'état des boîtiers (5a, 5b) reliés mécaniquement l'un à l'autre un mouvement de réglage d'un des éléments de commande (8a, 8b) entre sa position initiale et sa position de fonctionnement s'accompagne d'un mouvement de réglage correspondant de l'autre élément de commande (8b, 8a), de préférence, qu'une transmission (14), en particulier transmission par engrenages, est prévue, qui dans l'état des boîtiers (5a, 5b) reliés mécaniquement l'un à l'autre provoque un réglage synchrone des deux éléments de commande (8a, 8b) entre leur position initiale et leur position de fonctionnement, mieux encore, qu'un composant de transmission de la transmission (14), en particulier une roue dentée ou un segment de roue dentée de la transmission (14), est relié ou peut être relié de manière solidaire en rotation à un élément d'actionnement (15).

10. Connecteur stérile selon l'une quelconque des revendications précédentes, **caractérisé en ce que** le mouvement de réglage de l'élément de commande (8a, 8b) respectif entre sa position initiale et sa position de fonctionnement est un mouvement rotatif ou un mouvement linéaire.

11. Connecteur stérile selon l'une quelconque des revendications précédentes, **caractérisé en ce que** le mouvement de réglage de l'élément de commande (8a, 8b) respectif entre sa position initiale et sa position de fonctionnement est limité dans la position initiale et/ou position de fonctionnement par l'intermédiaire d'une butée (16a, 16b, 17a, 17b).

12. Connecteur stérile selon l'une quelconque des revendications précédentes, **caractérisé en ce que** l'entrée de fluide (11a) d'un des boîtiers (5a) et la sortie de fluide (12b) de l'autre boîtier (5b) peut être reliée ou est reliée à un tuyau (18a, 18b) ou tube, en particulier par l'intermédiaire d'un raccord emboîtable, de préférence, que l'entrée de fluide (11a) d'un des boîtiers (5a) est accouplée fluidiquement par l'intermédiaire du tuyau (18a) ou du tube à un récipient de liquide (2), en particulier à un récipient pour micro-quantités de liquide, pour la distribution d'un milieu liquide, en particulier biologique, et/ou que la sortie de fluide (12b) de l'autre boîtier (5b) est accouplée fluidiquement à une installation de bioprocédé (3), en particulier un bioréacteur, par l'intermédiaire du tuyau (18b) ou du tube.

13. Connecteur stérile selon l'une quelconque des revendications précédentes, **caractérisé en ce que** les deux boîtiers (5a, 5b) peuvent être reliés l'un à l'autre par coopération de formes, en particulier peuvent être emboîtés ou peuvent être encliquetés l'un avec l'autre.

14. Dispositif d'accouplement d'un connecteur stérile (1) pour le transfert stérile d'un milieu liquide, en particulier biologique, à partir d'un récipient de liquide (2) dans une installation de bioprocédé (3), en particulier dans un bioréacteur, en particulier connecteur stérile (1) selon l'une quelconque des revendications précédentes,
dans lequel le dispositif d'accouplement (4a, 4b) présente un boîtier (5a, 5b) avec un espace intérieur de boîtier (6a, 6b) et un élément de commande (8a, 8b) monté réglable dans l'espace intérieur de boîtier (6a, 6b), formant un canal (7a, 7b), dans lequel le canal (7a, 7b) s'étend à partir d'une ouverture d'entrée d'élément de commande (9a, 9b) vers une ouverture de sortie d'élément de commande (10a, 10b) à travers l'élément de commande (8a, 8b),
dans lequel le boîtier (5a, 5b) du dispositif d'accouplement (4a, 4b) peut être relié mécaniquement au boîtier (5b, 5a) d'un autre dispositif d'accouplement (4b, 4a) du connecteur stérile (1),
dans lequel le boîtier (5a, 5b) présente une entrée de fluide (11a, 11b) et une sortie de fluide (12a, 12b), qui relient l'espace intérieur de boîtier (6a, 6b) à l'environnement, dans lequel dans l'état des boîtiers (5a, 5b) reliés mécaniquement la sortie de fluide (12a, 12b) du boîtier (5a, 5b) du dispositif d'accouplement (4a, 4b) et l'entrée de fluide (11b, 11a) du boîtier (5b, 5a) de l'autre dispositif d'accouplement (4b, 4a) peuvent être amenés en coïncidence l'une avec l'autre,
dans lequel dans l'état des boîtiers (5a, 5b) reliés mécaniquement l'un à l'autre l'élément de commande (8a, 8b) est réglable à partir d'une position initiale, dans laquelle une liaison fluidique entre le canal (7a, 7b) et l'ouverture d'entrée d'élément de commande (9a, 9b) d'une part et entre le canal (7a, 7b) et l'ouverture de sortie d'élément de commande (10a, 10b) d'autre part est bloquée, dans une position de fonctionnement, dans laquelle l'ouverture d'entrée d'élément de commande (9a, 9b) est en liaison fluidique avec l'entrée de fluide (11a, 11b) et l'ouverture de sortie d'élément de commande (10a, 10b) avec la sortie de fluide (12a, 12b),
**caractérisé en ce**
**que** dans la position initiale le canal (7a, 7b) respectif s'étend dans une zone de l'espace intérieur de boîtier (6a, 6b) étanchéifiée hermétiquement par rapport à l'environnement du boîtier (5a, 5b).

15. Ensemble d'emballage avec un emballage (20, 22) et avec au moins un, de préférence un seul, dispositif d'accouplement (4a, 4b) selon la revendication 14, emballé de manière stérile dans celui-ci ou un connecteur stérile (1) selon l'une quelconque des revendications 1 à 13 emballé de manière stérile dans celui-ci.

16. Ensemble d'emballage selon la revendication 15, **caractérisé en ce que** l'élément de commande (8a, 8b) du dispositif d'accouplement (4a, 4b) emballé de manière stérile dans l'emballage (20, 22) est dans sa position initiale et qu'en outre un récipient de liquide (2), en particulier rempli, qui est accouplé fluidiquement au dispositif d'accouplement (4a) est emballé de manière stérile, ou une installation de bioprocédé (3), qui est accouplée fluidiquement au dispositif d'accouplement (4b), est emballée de manière stérile dans l'emballage (20, 22).

17. Utilisation d'un connecteur stérile (1) emballé de manière stérile selon l'une quelconque des revendications 1 à 13, d'un dispositif d'accouplement (4a, 4b) selon la revendication 14 emballé de manière stérile et/ou d'un ensemble d'emballage (19, 21) selon la revendication 15 ou 16 pour le transfert stérile d'un milieu liquide, en particulier biologique, à partir d'un récipient de liquide (2) dans une installation de bioprocédé (3), en particulier dans un bioréacteur.

18. Utilisation selon la revendication 17, **caractérisée en ce qu'**au moins un dispositif d'accouplement, en particulier les deux dispositifs d'accouplement (4a, 4b), de préférence également le récipient de liquide (2) et/ou l'installation de bioprocédé (3) et/ou le ou les tuyaux (18a, 18b) ou tubes, en particulier en tant que composant à usage unique, sont conçus au moins en partie, de préférence au moins principalement, à partir d'une matière plastique.
